# EUROPÄISCHE PATENTANMELDUNG

(11) **EP 0 672 661 A1**
(43) Veröffentlichungstag der Anmeldung: **20.09.1995**
(21) Anmeldenummer: 95103333.1
(22) Anmeldetag: 08.03.1995
(51) Int. Cl.: C07D 239/46, C07D 239/54, C07D 473/34, C07D 473/18, C07D 233/92, C07D 521/00, C08G 69/06, C07H 21/00, C08G 69/10

(54) **Substituierte N-Ethyl-Glycinderivate zur Herstellung von PNA und PNA-/DNA-Hybriden**

(30) Priorität: 14.03.1994 DE 4408534
(71) Anmelder: HOECHST AKTIENGESELLSCHAFT, D-65929 Frankfurt am Main (DE)
(72) Erfinder: Breipohl, Gerhard, Dr., D-60529 Frankfurt (DE); Uhlmann, Eugen, Dr., D-61479 Glashütten (DE); Knolle, Jochen, Dr., D-65830 Kriftel (DE)

(57) **Zusammenfassung**

Es werden N-Ethyl-Glycinderivate der Formel I 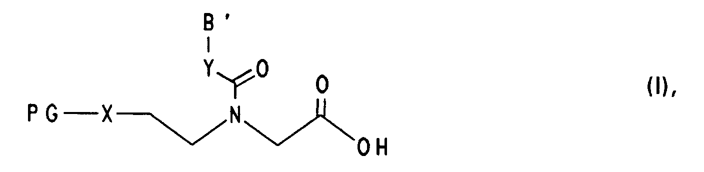
worin PG für eine gegen schwache Säuren labile Aminoschutzgruppe vom Urethantyp oder vom Trityl-Typ, X für NH, O oder S, Y für CH₂, NH oder O, und B' für in der Nucleotidchemie übliche Basen stehen, deren exocyclische Amino bzw. Hydroxygruppen durch geeignete bekannte Schutzgruppen geschützt sind, oder für Basenersatzverbindungen stehen und deren Salze mit tert. organischen Basen sowie ein Verfahren zu deren Herstellung beschrieben. Die N-Ethyl-Glycinderivate der Formel I finden Anwendung bei der Herstellung von PNA und PNA-/DNA-Hybriden.

## Beschreibung

Die vorliegende Erfindung betrifft neue substituierte N-Ethyl-Glycinderivate zur Herstellung von PNA und PNA-/DNA-Hybriden wie in der gleichzeitig eingereichten Anmeldung "Peptid-Oligonucleotid-Derivate, deren Herstellung und Verwendung" (HOE 94/F 057, DE-P 44 08 534.6) beschrieben.

Peptide bzw. Polyamide Nucleic Acids (PNA) sind DNA-analoge Verbindungen in denen das Deoxyribose-Phosphat-Gerüst durch ein Peptid-Oligomer ersetzt wurde. Die bisher in der Literatur (z. B. Michael Egholm, Peter E. Nielsen, Rolf H. Berg and Ole Buchardt, Science 1991, 254, 1497-1500; Ole Buchardt, Michael Egholm, Peter E. Nielsen and Rolf H. Berg, WO 92/20702) beschriebenen Synthesen verwenden als temporäre Schutzgruppe der Aminogruppe des Monomers die säurelabile tert.-Butyloxycarbonyl (Boc)-Schutzgruppe, die durch mittelstarke Säuren, wie z.B. Trifluoressigsäure abgespalten wird. Die Festphasensynthese von Oligomeren folgt dabei den üblichen Peptidsyntheseverfahren, wie sie z.B. von Merrifield (B. Merrifield, J. Am. Chem. Soc., 1963, 85, 2149) beschrieben wurde. Die Abspaltung des PNA-Oligomeres erfolgt dabei mit einer starken Säure, üblicherweise mit flüssigem Fluorwasserstoff.

Die wiederholte Behandlung mit Trifluoressigsäure und die anschließende Spaltung mit Fluorwasserstoff ist mit der Synthese von gemischten PNA/DNA-Sequenzen nicht kompatibel, da unter diesen Bedingungen die nucleosidische Bindung nicht stabil ist. Insbesondere werden die Purinnucleotide Desoxyguanosin und Desoxyadenosin mit starken Säuren an der N-glykosidischen Bindung rasch gespalten. Weiterhin wäre für die Synthese von derartigen Molekülen eine Verwendung von den üblichen DNA-Synthesizern und einer weitgehenden Beibehaltung der in diesen Geräten verwendeten Chemie besonders wünschenswert. Dies gilt auch für die Herstellung von PNA-Sequenzen mit der Hilfe solcher Geräte.

Ziel der Erfindung ist es daher, Glycinderivate zur Verfügung zu steilen, die einen einfachen Aufbau von PNA und PNA-/DNA-Hybriden und die Verwendung von Syntheseautomaten ermöglichen.

Für diesen Zweck geeignete Substanzen sind die Verbindungen der Formel I 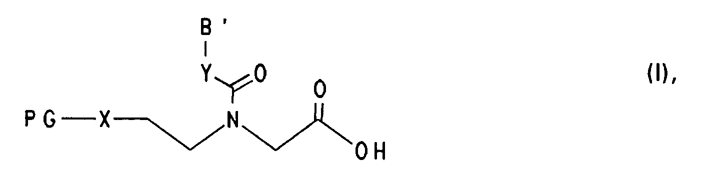
worin
- PG: für eine gegen schwache Säuren labile Aminoschutzgruppe vom Urethantyp, wie z.B. 1-(1-Adamantyl)1-methylethoxycarbonyl (Adpoc), 1-(3,5-di-tert.-butylphenyl)1-methylethoxycarbonyl (t-Bumeoc) und 1-Methyl-1-(4-biphenyl)ethyloxycarbonyl (Bpoc), 3,5-Dimethoxyphenyl-2-propyl-2-oxycarbonyl (Ddz) oder vom Trityl-Typ, wie Triphenylmethyl (Trt), (4-Methoxyphenyl)diphenylmethyl (Mmt), (4-Methylphenyl)diphenylmethyl (Mtt), Di-(4-methoxyphenyl)phenylmethyl (Dmt) und 9-(9-Phenyl)xanthenyl (Pixyl),
- X: für NH, O oder S, bevorzugt für NH oder O,
- Y: für CH₂, NH oder O, bevorzugt für CH₂, und
- B': für in der Nucleotidchemie übliche Basen, beispielsweise für natürliche Basen, wie Adenin, Cytosin, Guanin, Thymin und Uracil, oder unnatürliche Basen, wie Purin, 2.6-Diaminopurin, 7-Deazaadenin, 7-Deazaguanin, N⁴N⁴-Ethanocytosin, N⁶N⁶-Ethano-2.6-diaminopurin, 5-Methylcytosin,5-(C₃-C₆)-Alkinyl-uracil, 5-(C₃-C₆)-Alkinyl-cytosin, 5-Fluor-uracil und Pseudoisocytosin, deren exocyclische Amino bzw. Hydroxygruppen durch geeignete bekannte Schutzgruppen, wie die Benzoyl-, Isobutanoyl-, Acetyl-, Phenoxyacetyl-, 4-(t-Butyl)-benzoyl-, 4-(t-Butyl)-Phenoxyacetyl-, 4-(Methoxy)-benzoyl-, 2-(4-Nitrophenyl)ethyl-oxycarbonyl-, 2-(2,4-Dinitrophenyl)ethyl-oxycarbonyl-, 9-Fluorenylmethoxycarbonyl-, Diphenylcarbamoyl- oder Formamidin-Gruppe, bevorzugt die Benzoyl-, Isobutanoyl-, Acetyl-, Phenoxyacetyl-, 4-(t-Butyl)-benzoyl-, 4-(Methoxy)-benzoyl-Gruppe, und auch für Guanin durch eine Kombination von 2-N-Acetyl mit 6-O-Diphenylcarbamoyl geschützt sind, oder für Basenersatzverbindungen wie z.B. Imidazol, Triazol oder Nitro-Imidazol stehen,
sowie deren Salze, bevorzugt deren Salze mit tert. organischen Basen, wie z.B. Triethylamin oder Pyridin.

Verbindungen der Formel I mit Y in der Bedeutung CH₂ können beispielsweise dadurch erhalten werden, daß man
eine Verbindung der Formel II 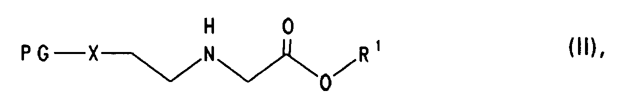
worin
- PG und X: wie oben definiert sind, und
- R¹: Wasserstoff oder eine Esterschutzgruppe, wie z.B. Methyl, Ethyl, Butyl oder 2-(Methoxy-ethoxy)-ethyl, bedeutet,
mit einer Verbindung der Formel III 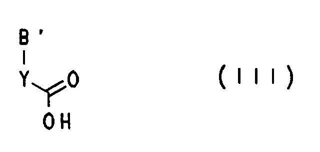
worin
- B': wie oben definiert ist und
- Y: für CH₂ steht,
bei 0 - 45°C, bevorzugt bei Raumtemperatur, in einem geeigneten Lösungsmittel, wie z.B. DMF, Acetonitril, Dichlormethan oder Mischungen dieser Lösungsmittel unter Verwendung eines in der Peptidchemie üblichen Kupplungsreagenzes, wie z.B. Carbodiimiden, Phosphonium-Reagenzien, Uronium-Reagenzien, Säurehalogeniden, aktivierten Estern zu einer Verbindung der Formel IV 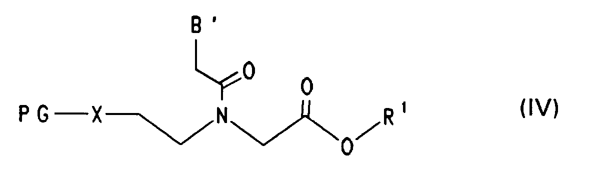
worin
PG, X, B' und R¹ wie oben definiert sind,
umsetzt und anschließend diese Verbindung durch Abspaltung der Esterschutzgruppe R¹ unter schwach alkalischen Bedingungen mit Alkalilauge, wie z.B. NaOH, LiOH, KOH, oder durch tertiäre Aminverbindungen in Wasser, wie z.B. Triethylamin, oder auch enzymatisch mit Hilfe von Esterasen oder Lipasen bei 0 - 50°C, bevorzugt bei Raumtemperatur, in einem geeigneten Lösungsmittel, wie Dioxan, Wasser, Tetrahydrofuran, Methanol, Wasser oder Gemischen aus diesen Lösungsmitteln in eine Verbindung der Formel I überführt.

In der Peptidsynthese übliche Aktivierungsmethoden sind z.B. in Houben-Weyl, Methoden der organischen Chemie, Band 15/2, Georg Thieme Verlag Stuttgart 1994, oder weitere Reagenzien, wie z.B. BOP (B. Castro, J.R. Dormoy, G. Evin and C. Selve, Tetrahedron Lett. 1975, 1219-1222), PyBOP (J. Coste, D. Le-Nguyen und B. Castro, Tetrahedron Lett. 1990, 205-208), BroP (J. Coste, M.-N. Dufour, A. Pantaloni und B. Castro, Tetrahedron Lett. 1990, 669-672), PyBroP (J. Coste, E. Frerot, P. Jouin und B. Castro, Tetrahedron Lett. 1991, 1967-1970) und Uronium-Reagenzien, wie z.B. HBTU (V. Dourtoglou, B. Gross, V. Lambropoulou, C. Zioudrou, Synthesis 1984, 572-574), TBTU, TPTU, TSTU, TNTU, (R. Knorr, A. Trzeciak, W. Bannwarth and D. Gillessen, Tetrahedron Letters 1989, 1927-1930), TOTU (EP-A-0 460 446), HATU (L.A. Carpino, J. Am. Chem. Soc. 1993, 115, 4397-4398), HAPyU, TAPipU (A. Ehrlich, S. Rothemund, M. Brudel, M. Beyermann, L.A. Carpino und M. Bienert, Tetrahedron Lett. 1993, 4781-4784), BOI (K. Akaji, N. Kuriyama, T. Kimura, Y. Fujiwara und Y. Kiso, Tetrahedron Lett. 1992, 3177-3180) oder Säurechloride bzw. Säurefluoride (L. A. Carpino, H. G. Chao, M. Beyermann and M. Bienert, J. Org. Chem., 56(1991), 2635; J.-N. Bertho, A. Loffet, C. Pinel, F. Reuther and G. Sennyey in E. Giralt and D. Andreu (Eds.) Peptides 1990, Escom Science Publishers B. V.1991, pp. 53-54; J. Green und K. Bradley, Tetrahedron 1993, 4141-4146), 2,4,6-Mesitylensulfonyl-3-nitro-1,2,4-triazolid (MSNT) (B. Blankemeyer-Menge, M. Nimitz und R. Frank, Tetrahedron Lett. 1990, 1701-1704), 2,5-Diphenyl-2,3-dihydro-3-oxo-4-hydroxythiophendioxid (TDO) (R. Kirstgen, R.C. Sheppard, W. Steglich, J. Chem. Soc. Chem. Commun. 1987, 1870-1871) oder aktivierte Ester (D. Hudson) Peptide Res. 1990, 51 - 55) sind in den jeweiligen Literaturstellen beschrieben.

Bevorzugt ist die Verwendung von Carbodiimiden, z.B. Dicyclohexylcarbodiimid oder Diisopropylcarbodiimid. Bevorzugt verwendet werden ebenfalls Phosphonium-Reagenzien, wie z.B. PyBOP oder PyBroP, Uronium-Reagenzien, wie z.B. HBTU, TBTU, TPTU, TSTU, TNTU, TOTU oder HATU, BOI oder Säurechloride bzw. Säurefluoride.

Für die Synthese der Verbindungen der Formel II werden Aminoethylglycin, Hydroxyethylglycin, Mercaptoethylglycin oder deren entsprechende Ester mit der entsprechenden gegen schwache Säuren labilen Schutzgruppe versehen. Die Einführung der gegen schwache Säuren labilen Schutzgruppe erfolgt dabei mit an sich literaturbekannten, zum Teil modifizierten Verfahren. Geeignete Reagenzien sind z.B. t-Bumeoc-fluorid, Adpoc-azid, Bpoc-azid, Ddz-(phenyl)carbonat, Trt-Cl, Mtt-Cl, Mmt-Cl, Dmt-Cl, Pixyl-Cl. Bei dieser Umsetzung kann die Löslichkeit des Aminoethylglycins unter gleichzeitigem Schutz der Säurefunktion durch Umsetzung mit üblichen Silylierungsreagenzien, wie z.B. Bis-Trimethylsilylacetamid, verbessert werden. Die Abspaltung dieser temporären Schutzgruppe erfolgt nach der Umsetzung mit den Schutzgruppenreagenzien durch Zugabe von Wasser oder Alkoholen zur Reaktionsmischung. Das als Ausgangsmaterial verwendete Aminoethylglycin oder der entsprechende Aminoethylglycinester werden nach literaturbekannter Methode (E.P. Heimer, H.E. Gallo-Torres, A.M. Felix, M. Ahmad, T.J. Lambros, F. Scheidl and J. Meienhofer, Int. J. Peptide Protein Res. 23, 1984, 203-211) hergestellt.

Ein weiteres Verfahren zur Herstellung von Aminoethylglycin besteht in der reduktiven Aminierung von Glyoxylsäure mit Ethylendiamin und ist in der gleichzeitig eingereichten Anmeldung mit dem Titel "Verfahren zur Herstellung von Aminoethylglycin" (HOE 94/F 061, DE-P 44 08 530.3) beschrieben.

Die Synthese von 2-Hydroxyethylglycin bzw. 2-Mercaptoethylglycin erfolgt z.B. durch reduktive Aminierung von Glyoxylsäure oder Glyoxylsäureestern mit Aminoethanol oder Cysteamin mit Wasserstoff an Palladium auf Kohle oder im Falle von 2-Mercaptoethylglycin bevorzugt mit Natriumcyanoborhydrid oder Natrium-triacetoxy-borhydrid als Reduktionsmittel.

Die sich von 2-Mercaptoethylglycin ableitende Verbindung der Formel I kann auch dadurch erhalten werden, daß man zunächst 2-Mercaptoethylamin oder dessen Hydrochlorid in Essigsäure oder Essigsäure/Wasser Gemischen mit dem entsprechenden Triphenylderivat, wie z. B. deren Halogeniden oder Alkoholen, umsetzt. Die Reaktion erfolgt unter den sauren Bedingungen über das entsprechende Tritylkation bevorzugt am Schwefel des 2-Mercaptoethylamins. Bevorzugt ist die Verwendung von (4-Methoxyphenyl)diphenylmethyl-chlorid für die Einführung der Mmt-Gruppe oder von Di-(4-methoxyphenyl)phenylmethylchlorid für die Einführung der Dmt-Gruppe. Anschließend wird die Aminogruppe durch Umsetzung mit Halogenessigsäureestern, bevorzugt Bromessigsäureestern mit einer organischen Hilfsbase wie z.B. Diisopropylethylamin in einem geeigneten Lösungsmittel, wie z.B. DMF alkyliert. Die hierbei entstehende Verbindung der Formel II mit PG = eine Schutzgruppe vom Trityltyp, X = S und R¹ = eine Esterschutzgruppe kann dann in der vorgehend beschriebenen Weise zu den entsprechenden Verbindungen der Formel I umgesetzt werden.

Die Nucleobasen-Essigsäurederivate der Formel III können durch Alkylierung der entsprechenden Nucleobasen oder der in der exocyclischen Hydroxy- oder Aminofunktion geschützten Nucleobasen mit Chloressigsäure, Bromessigsäure, Jodessigsäure oder deren Estern erhalten werden. Gegebenenfalls werden dabei zur selektiven Alkylierung zusätzlich temporäre Schutzgruppen an der Nucleobase eingeführt. Als Schutzgruppe für die Nucleobasen können alle mit der gegen schwache Säuren labilen Schutzgruppe PG kompatible Schutzgruppen verwendet werden. Für die exocyclische Aminofunktion werden bevorzugt Schutzgruppen, wie die Benzoyl-, Isobutanoyl-, Acetyl-, Phenoxyacetyl-, 4-(t-Butyl)-benzoyl-, 4-(t-Butyl)-Phenoxyacetyl-, 4-(Methoxy)-benzoyl-, 2-(4-Nitrophenyl)ethyl-oxycarbonyl-, 2-(2,4-Dinitrophenyl)ethyl-oxycarbonyl-, 9-Fluorenylmethoxycarbonyl-, Diphenylcarbamoyl- oder Formamidin-Gruppe verwendet. Insbesondere bevorzugt sind die Benzoyl-, Isobutanoyl-, 4-(t-Butyl)-benzoyl-, 2-(4-Nitrophenyl)ethyl-oxycarbonyl-, 2-(2,4-Dinitrophenyl)ethyl-oxycarbonyl-, 9-Fluorenylmethoxycarbonyl-, 4-(Methoxy)-benzoyl- oder para-(t-Butyl)-Phenoxyacetyl-, para-Nitrophenyl-2-ethyl-oxycarbonyl-Gruppe und für Guanin eine Kombination der 2-N-Acetyl mit der 6-O-Diphenylcarbamoyl-Gruppe.

Ein alternatives Verfahren zur Herstellung der Verbindungen der Formel I mit Y in der Bedeutung CH₂ besteht darin, daß man
eine Verbindung der Formel II 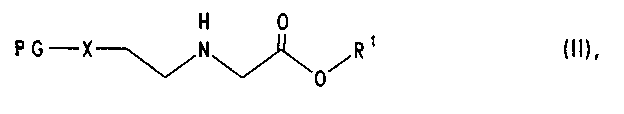
worin
- PG und X: wie oben definiert sind und
- R¹: Wasserstoff oder eine temporäre Silylschutzgruppe, wie z.B. Trimethylsilyl, bedeutet,
mit einer Verbindung der Formel V

B' - CH₂ - CO - R² (V),

worin
- B': wie oben definiert ist und
- R²: Halogen, wie z.B. Fluor, Chlor oder Brom, oder den Rest eines Aktivesters, wie z.B. OBt, OObt, OPfp, ONSu, bedeutet,
bei 0 - 40°C, bevorzugt 20 - 30°C, in einem geeigneten Lösungsmittel, wie zum Beispiel DMF, NMP, Acetonitril, Dichlormethan oder Mischungen dieser Lösungsmittel umsetzt, wobei gegebenenfalls der temporäre Schutz der Säurefunktion in der Verbindung der Formel II durch Umsetzung mit üblichen Silylierungsreagenzien, wie z.B. Bis-Trimethylsilyacetamid, und die Abspaltung dieser temporären Schutzgruppe - nach der Umsetzung mit der Verbindung der Formel V - durch Zugabe von Wasser oder Alkoholen zur Reaktionsmischung erfolgen kann.

Ein weiteres alternatives Verfahren zur Herstellung der Verbindungen der Formel I mit Y in der Bedeutung CH₂ besteht darin, daß man eine Verbindung der Formel II 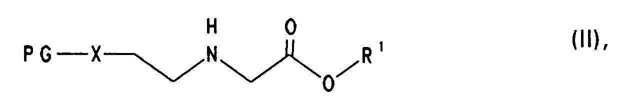
worin
- PG und X: wie oben definiert sind und
- R¹: eine Esterschutzgruppe, wie z.B. Methyl, Ethyl, Butyl, 2-(Methoxy-ethoxy)-ethyl etc., bedeutet,
mit einem Halogen-essigsäurederivat, wie z.B. Chloressigsäurechlorid, Bromessigsäurebromid, Bromessigsäurechlorid oder Jodessigsäurechlorid, in einem geeigneten Lösungsmittel, wie z.B. Tetrahydrofuran, Dichlormethan oder DMF, mit einer Hilfsbase, wie z.B. Triethylamin, N-Ethylmorpholin oder Diisopropylethylamin, zur Verbindung der Formel VI 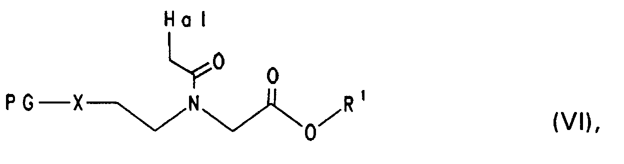
worin
- Hal: Cl, Br, I, bevorzugt Br oder Cl, ganz besonders bevorzugt Cl, bedeutet und
- PG, X und R¹: wie oben definiert sind,
umsetzt,
diese Zwischenverbindung der Formel VI mit der gegebenenfalls geschützten Nucleobase B' und einer Hilfsbase, z.B. Kaliumcarbonat, in einem geeigneten Lösungsmittel, z.B. DMF oder NMP, zur Verbindung der Formel IV 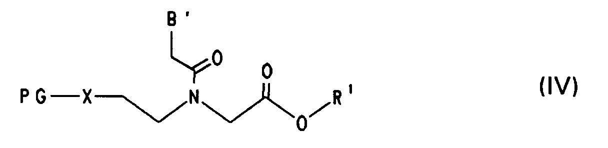
umsetzt,
anschließend diese Verbindung durch Abspaltung der Esterschutzgruppe R¹ mit Alkalilauge, wie z.B. NaOH, LiOH, KOH, oder auch enzymatisch mit Hilfe von Esterasen oder Lipasen bei 0 - 50°C, bevorzugt bei Raumtemperatur, in einem geeigneten Lösungsmittel, wie Dioxan, Wasser, Tetrahydrofuran, Methanol, Wasser oder Gemischen aus diesen Lösungsmitteln in eine Verbindung der Formel I überführt.

Verbindungen der Formel I mit Y in der Bedeutung O oder NH werden dadurch erhalten, daß man
eine Verbindung der Formel II 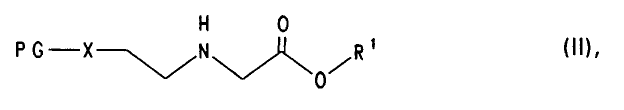
worin
- PG und X: wie oben definiert sind und
- R¹: eine Esterschutzgruppe, wie z.B. Methyl, Ethyl, Butyl, 2-(Methoxy-ethoxy)-ethyl, bzw. eine temporäre Esterschutzgruppe, wie z.B. Trimethylsilyl, bedeutet,
mit einer Verbindung der Formel VII

B' - Y - CO - R³ (VII),

worin
- B': wie oben definiert ist,
- Y: O oder NH bedeutet und
- R³: Cl, ONp, ONSu bedeutet,
bei 0 - 40°C, bevorzugt 20 - 30°C, in einem geeigneten Lösungsmittel, wie DMF, Acetonitril oder Dichlormethan, oder Mischungen dieser Lösungsmittel umsetzt und anschließend die Esterschutzgruppe R¹ mit Alkalilauge, wie z.B. NaOH, LiOH, KOH, oder auch enzymatisch mit Hilfe von Esterasen oder lipasen bei 0 - 50°C, bevorzugt bei Raumtemperatur, in einem geeigneten Lösungsmittel, wie Dioxan, Wasser, Tetrahydrofuran, Methanol, Wasser oder Gemischen aus diesen Lösungsmitteln abspaltet.

Die als Ausgangsmaterial für die letztere Reaktion benötigten N-Amino-bzw. N-Hydroxy-Nucleobasen werden nach bekannten Verfahren wie z.B. bei K. Kohda, I. Kobayashi, K. Itano, S. Asano, Y. Kawazoe (Tetrahedron 49, 3947-3958 (1993)) beschrieben erhalten und dann mit Phosgen oder Chlorameisensäureestern zu den Carbamaten oder Carbonaten der Formel VII umgesetzt.

Die für Aminosäuren verwendeten Abkürzungen entsprechen dem in der Peptidchemie üblichen drei Buchstaben-Code wie er in Europ. J. Biochem. 138, 9 (1984) beschrieben ist. Weitere verwendete Abkürzungen sind nachfolgend aufgelistet.
- Aeg: N-(2-Aminoethyl)glycyl, -NH-CH₂-CH₂-NH-CH₂-CO-
- Aeg(A^{MeOBz}): N-(2-Aminoethyl)-N-((9-(N⁶-4-methoxybenzoyl)-adenosyl)acetyl)-glycyl
- Aeg(C^{Bz}): N-(2-Aminoethyl)-N-((1-(N⁴-benzoyl)-cytosyl)acetyl)-glycyl
- Aeg(C^{MeOBz}): N-(2-Aminoethyl)-N-((1-(N⁴-4-methoxybenzoyl)-cytosyl)acetyl)-glycyl
- Aeg(C^{tBuBz}): N-(2-Aminoethyl)-N-((1-(N⁴-4-tert.butylbenzoyl)-cytosyl)acetyl)-glycyl
- Aeg(G^{iBu}): N-(2-Aminoethyl)-N-((9-(N²-isobutanoyl)-guanosyl)acetyl)-glycyl
- Aeg(G^{2-Ac,4-Dpc}): N-(2-Aminoethyl)-N-((9-(N²-acetyl-O⁴-diphenylcarbamoyl)guanosyl)glycyl
- Aeg(Im): N-(2-Amino)ethyl-N-((1-imidazolyl)acetyl)glycyl
- Aeg(Im^{4-Nitro}): N-(2-Amino)ethyl-N-((1-(4-nitro)imidazolyl)acetyl)glycyl
- Aeg(T): N-(2-Aminoethyl)-N-((1-thyminyl)acetyl)-glycyl
- Aeg(Triaz): N-(2-Amino)ethyl-N-((1-(1,2,4)-triazolyl)acetyl)glycyl
- Bnpeoc: 2,2-[Bis(4-nitrophenyl)]-ethoxycarbonyl)
- Boc: tert.-Butyloxycarbonyl
- BOI: 2-(Benzotriazol-1-yl)oxy-1,3-dimethyl-imidazolidinium-hexafluorphosphat
- BOP: Benzotriazolyl-1-oxy-tris(dimethylamino)-phosphonium-hexafluorphosphat
- BroP: Brom-tris(dimethylamino)-phosphonium-hexafluorphosphat
- BSA: N,O-Bis-(trimethylsilyl)-acetamid
- But: tert.-Butyl
- Bz: Benzoyl
- Bzl: Benzyl
- Cl-Z: 4-Chlor-benzyloxycarbonyl
- CPG: Controlled Pore Glas
- DBU: 1,8-Diazabicyclo[5.4.0]undec-7-en
- DCM: Dichlormethan
- Ddz: 3.5-Dimethoxyphenyl-2-propyl-2-oxycarbonyl
- DMF: Dimethylformamid
- Dmt: Di-(4-methoxyphenyl)phenylmethyl,
- Dnpeoc: 2-(2,4-Dinitrophenyl)-ethoxycarbonyl
- Dpc: Diphenylcarbamoyl
- FAM: Fluorescein-Rest
- Fm: 9-Fluorenylmethyl
- Fmoc: 9-Fluorenylmethyloxycarbonyl
- H-Aeg-OH: N-(2-Aminoethyl)glycin
- HAPyU: O-(7-Azabenzotriazol-1-yl)-1,1,3,3-bis(tetramethylen)uronium-hexafluorphosphat
- HATU: O-(7-Azabenzotriazol-1-yl)-1,1,3,3-tetramethyluronium-hexafluorphosphat
- HBTU: O-(Benzotriazol-1-yl)-1,1,3,3-tetramethyluronium-hexafluorphosphat
- HOBt: 1-Hydroxybenzotriazol
- HONSu: N-Hydroxysuccinimid
- HOObt: 3-Hydroxy-4-oxo-3,4-dihydrobenzotriazin
- iBu: Isobutanoyl
- MeOBz: 4-Methoxybenzoyl
- Mmt: 4-Methoxytriphenylmethyl
- Moz: 4-Methoxybenzyloxycarbonyl
- MSNT: 2,4,6-Mesitylensulfonyl-3-nitro-1,2,4-triazolid
- Mtt: 4-Methylphenyl)diphenylmethyl
- NBA: Nitrobenzylalkohol
- NMP: N-Methylpyrrolidin
- Oeg: N-(2-Oxyethyl)glycyl, -O-CH₂-CH₂-NH-CH₂-CO-
- Pixyl: 9-(9-Phenyl)xanthenyl
- PyBOP: Benzotriazolyl-1-oxy-tripyrrolidinophosphonium-hexafluorphosphat
- PyBroP: Brom-tripyrrolidinophosphonium-hexafluorphosphat
- TAPipU: O-(7-Azabenzotriazol-1-yl)-1,1,3,3-bis(pentamethylen)uronium-tetrafluoroborat
- TBTU: O-(Benzotriazol-1-yl)-1,1,3,3-tetramethyluronium-tetrafluoroborat
- tBu: tert.-Butyl
- tBuBz: 4-tert.Butylbenzoyl
- TDBTU: O-(3,4-Dihydro-4-oxo-1,2,3-benzotriazin-3-yl)-1,1,3,3-tetramethyluronium-tetrafluoroborat
- TDO: 2,5-Diphenyl-2,3-dihydro-3-oxo-4-hydroxythiophendioxid
- Teg: N-(2-Thioethyl)glycyl, -S-CH₂-CH₂-NH-CH₂-CO-
- TFA: Trifluoressigsäure
- THF: Tetrahydrofuran
- TNTU: O-[(5-Norbornen-2,3-dicarboximido]-1,1,3,3-tetramethyluronium-tetrafluoroborat
- TOTU: O-[(Cyano(ethoxycarbonyl)methylen)amino]-1,1,3,3-tetramethyluronium-tetrafluoroborat
- TPTU: O-(1,2-Dihydro-2-oxo-1-pyridyl)-1,1,3,3'-tetramethyluronium-tetrafluoroborat
- Trt: Trityl
- TSTU: O-(N-Succinimidyl)-1,1,3,3-tetramethyluronium-tetrafluoroborat
- Z: Benzyloxycarbonyl
- MS(ES⁺): Elektrospray Massenspektrum (Positiv Ion)
- MS(ES⁻): Elektrospray Massenspektrum (Negativ Ion)
- MS(DCI): Desorption Chemical Ionisation Massenspektrum
- MS(FAB): Fast-Atom-Bombardment-Massenspektrum

### Beispiele:

### Beispiel 1

### Herstellung von N-(Hydroxyethyl)glycin H-Oeg-OH

46 g Glyoxylsäure-Monohydrat werden in 1000 ml Wasser gelöst und dann unter Kühlung und Rühren 30.2 ml 2-Aminoethanol dazugegeben. Die Mischung wird mit 10 g Katalysator (10% Pd/C) versetzt und im Autoklaven bei 10 bar Wasserstoffdruck und Raumtemperatur hydriert. Zur Aufarbeitung wird vom Katalysator abgesaugt und das Filtrat im Vakuum zur Trockene eingeengt. Der Rückstand wird zweimal mit wenig Toluol nachdestilliert. Dieses Rohprodukt wird mit 250 ml heißem Methanol digeriert, warm abgesaugt, mit wenig Methanol nachgewaschen und dann im Exsiccator getrocknet.
Ausbeute: 49.56 g
Fp.: 178-180°C, Zers.
R_{f}= 0.36 (n-Butanol/Essigsäure/Wasser/Ethylacetat 1:1:1:1)
MS(DCI): 120 (M+H)⁺

### Beispiel 2

### Synthese von N-(9-Fluorenylmethoxycarbonyl)-N-(hydroxyethyl)glycin H-Oeg(Fmoc)-OH

10.08 g Natriumhydrogencarbonat werden in 150 ml Wasser gelöst und dann 7.15 g N-(Hydroxyethyl)glycin unter Rühren zugegeben. Nach ca. 5 min erhält man eine klare Lösung zu der 20.22 g Fmoc-ONSu in 300 ml Dioxan unter gutem Rühren zugetropft werden. Man rührt noch 3 h bei Raumtemperatur nach und läßt über Nacht bei 4°C stehen. Die Lösung wird filtriert und das Filtrat am Rotationsverdamfer im Vakuum eingeengt. Der Rückstand wird in 100 ml Wasser aufgenommen und durch portionsweise Zugabe von Kaliumhydrogensulfat-Lösung auf pH 2 gestellt. Dann extrahiert man mit dreimal 150 ml Ethylacetat, wäscht die organische Phase mit viermal 50 ml Wasser und trocknet über Natriumsulfat. Man filtriert vom Trockenmittel und engt das Filtrat am Rotationsverdamfer im Vakuum zur Trockene ein. Der Rückstand wird mit 200 ml Diisopropylether versetzt und verrieben, wobei Kristallisation eintritt. Man läßt über Nacht stehen, saugt den Niederschlag ab und kristallisiert aus Ethylacetat/Diisopropylether um. Das so erhaltene Produkt wird abgesaugt und im Exsiccator getrocknet.
Ausbeute: 15.8 g
Fp.: 114-116°C, Zers.
R_{f} = 0.62 (n-Butanol/Essigsäure/Wasser 3:1:1)
MS(ES⁺): 342 (M+H)⁺

### Beispiel 3

### Synthese von N-(9-Fluorenylmethoxycarbonyl)-N-(4-methoxyphenyl-diphenylmethoxyethyl)glycin Mmt-Oeg(Fmoc)-OH

14.74 g N-(Fluorenylmethoxycarbonyl)-N-(hydroxyethyl)glycin werden in 160 ml Pyridin gelöst und 13.31 g 4-Methoxyphenyl-diphenyl-methylchlorid zugegeben. Die Mischung wird 2h bei Raumtemperatur gerührt und über Nacht bei 4°C stehengelassen. Dann wird das Lösungsmittel im Vakuum am Rotationsverdampfer agezogen und der Rückstand in 300 ml Ethylacetat aufgenommen und mit dreimal 30 ml gesättigter wäßriger Bicarbonat-Lösung extrahiert. Dann wird noch mit dreimal 30 ml Wasser gewaschen und die organische Phase über Natriumsulfat getrocknet. Das Trockenmittel wird abfiltriert, das Filtrat auf ca 70 ml Volumen eingeengt und unter Rühren in 700 ml Diisopropylether eingetropft. Das ausgefallene Produkt wird abgesaugt und im Exsiccator getrocknet.
Ausbeute: 19.95 g
R_{f} = 0.45 (Ethylacetat/Methanol/Triethylamin = 60:40:1)
MS(FAB/MeOH/NBA): 658.3 (M+2Na)⁺

### Beispiel 4

### N⁴-(4-methoxybenzoyl)-cytosin

### Beispiel 4a:

Cytosin (11.1 g) wird in trockenem Pyridin (250 ml) suspendiert. Dann wird mit einer Spritze 4-Methoxybenzoylchlorid (17.06 g) tropfenweise zugegeben. Es entsteht eine fast klare Lösung aus der nach ca. 10 min ein Niederschlag ausfällt. Man rührt noch ca. 1.5 h bei Raumtemperatur nach und filtriert dann ab. Der Niederschlag ist das gewünschte Produkt.
Ausbeute: 20.5 g
MS(ES⁺): 246 (M+H)⁺
R_{f} = 0.77 (Dichlormethan:Isopropanol/8:2)

### Beispiel 4b:

Cytosin (2.8 g) wird in trockenem DMF (100 ml) suspendiert und mit Triethylamin (2 ml) versetzt. Dann wird mit einer Spritze 4-Methoxybenzoesäureanhydrid (7.2 g) tropfenweise zugegeben. Es entsteht eine fast klare Lösung aus der nach einiger Zeit ein Niederschlag ausfällt. Man rührt noch ca. 2 h bei Raumtemperatur nach und filtriert dann ab. Der Niederschlag ist das gewünschte Produkt.
Ausbeute: 5.9 g
MS(ES⁺): 246 (M+H)⁺
R_{f} = 0.77 (Dichlormethan:Isopropanol/8:2)

### Beispiel 5

### N⁴-(4-methoxybenzoyl)-N¹-methoxycarbonylmethylcytosin

N⁴-(4-methoxybenzoyl)-cytosin (12.25 g) wird in trockenem DMF (250 ml) suspendiert und Natriumhydrid (1.25 g) in Portionen zugegeben. Die Mischung wird kurz auf 60°C erhitzt bis die Wasserstoffentwicklung beendet ist. Bei Raumtemperatur wird anschließend mit einer Spritze Bromessigsäuremethylester (4.75 ml) tropfenweise zugegeben. Man rührt noch 1h bei Raumtemperatur nach und versetzt dann mit wenig Kohlendioxid in Methanol. Das Lösungsmittel wird im Vakuum abgezogen und der verbliebene Rückstand mit Dichlormethan versetzt und dann filtriert. Der Filterrückstand wird noch mit wenig Wasser gewaschen und dann im Vakuum getrocknet.
Ausbeute 12.86 g
Fp.: 219°C
MS(ES⁺): 318 (M+H)⁺
R_{f} = 0.71 (n-Butanol:Essigsäure:Wasser/3:1:1)

### Beispiel 6

### N⁴-(4-methoxybenzoyl)-N¹-carboxymethylcytosin

N⁴-(4-methoxybenzoyl)-N¹-methoxycarbonylmethylcytosin (12.5 g) wird in Wasser (100 ml) suspendiert und bei 0°C tropfenweise mit 2N wäßriger Natronlauge unter pH-Kontrolle (pH 12) versetzt bis der Methylester verseift ist. Der Fortgang der Reaktion wird mittels DC verfolgt. Dann wird die Reaktionslösung Filtriert und das Filtrat mit 2M Kaliumhydrogensulfat-Lösung auf pH 3 gestellt wobei das Produkt ausfällt. Man filtriert den Niederschlag ab, wäscht mit wenig kaltem Wasser nach und trocknet im Vakuum.
Ausbeute: 11.5 g
Fp.: 270-275°C, Zers.
MS(ES⁺): 304 (M+H)⁺
R_{f} = 0.53 (n-Butanol:Essigsäure:Wasser/3:1:1)

### Beispiel 7

### N⁴-(4-tert.-Butylbenzoyl)-cytosin

Cytosin (11.1 g) wird in trockenem DMF (250 ml) suspendiert und mit Triethylamin (15.4 ml) versetzt. Dann wird mit einer Spritze 4-tert.-Butyl-benzoylchlorid (18.6 ml) tropfenweise zugegeben. Man rührt noch ca. 4 h bei Raumtemperatur nach und gibt dann weiteres 4-tert.-Butyl-benzoylchlorid (3.7 ml) hinzu. Nach weiteren 2 h wird die Reaktion durch Zugabe von etwas Methanol gequencht. Man entfernt das Lösungsmittel im Vakuum und versetzt den Rückstand mit Dichlormethan und Wasser. Der dabei ausfallende Feststoff ist die gewünschte Substanz. Der Niederschlag wird abfiltriert und im Vakuum getrocknet.
Ausbeute: 15.7 g
MS(DCI): 272 (M + H)⁺
R_{f} = 0.55 (Dichlormethan:Methanol/9:1)

### Beispiel 8

### N⁴-(4-tert-Butylbenzoyl)-N¹-methoxycarbonylmethyl-cytosin

N⁴-(4-tert-Butylbenzoyl)-cytosin (14.96 g) wird in trockenem DMF (250 ml) suspendiert, Natriumhydrid (1.32 g) in Portionen zugegeben und die Mischung 1 h bei Raumtemperatur gerührt bis die Wasserstoffentwicklung beendet ist. Bei Raumtemperatur wird anschließend mit einer Spritze Bromessigsäuremethylester (5.2 ml) tropfenweise zugegeben. Man rührt noch 1 h bei Raumtemperatur nach und versetzt dann mit Methanol (10 ml). Das Lösungsmittel wird im Vakuum abgezogen und der verbliebene Rückstand zwischen Dichlormethan und Wasser verteilt. Die organische Phase wird mit Wasser gewaschen, über Natriumsulfat getrocknet und nach Filtration im Vakuum eingeengt. Das so erhaltene Rohprodukt wird aus Isopropanol umkristallisiert und dann im Vakuum getrocknet.
Ausbeute: 8.0 g
Fp.: 189-193°C, Zers.
MS(DCI): 344 (M+H)⁺
R_{f} = 0.72 (Dichlormethan:Methanol/9:1)

### Beispiel 9

### N⁴-(4-tert-Butylbenzoyl)-N¹-carboxymethylcytosin

N⁴-(4-tert-Butylbenzoyl)-N¹-methoxycarbonylmethylcytosin (8.0 g) wird in einer Mischung von Dioxan (50 ml) und Wasser (10 ml) gelöst und unter Rühren bei Raumtemperatur 2N wäßrige Natronlauge tropfenweise zugegeben (pH 11-12). Wenn die Verseifung des Methylesters beendet ist, -DC-Kontrolle- wird die Reaktionslösung mit 2M Kaliumhydrogensulfat-Lösung auf pH 3 gestellt. Der ausgefallene Niederschlag wird abgesaugt. Diese Rohprodukt wird in Natriumcarbonat-Lösung gelöst und erneut durch Zugabe von 2M Kaliumhydrogensulfat-Lösung ausgefällt. Das Produkt wird abgesaugt, mit wenig Wasser nachgewaschen und im Vakuum getrocknet.
Ausbeute: 6.62 g
Fp.: ab 285°C, Zers.
MS(DCI): 330 (M+H)⁺
R_{f} = 0.2 (Dichlormethan:Methanol/9:1)

### Beispiel 10

### N⁴-(Benzoyl)-cytosin

Cytosin (5.55 g) wird in trockenem Pyridin (100 ml) suspendiert. Dann wird mit einer Spritze Benzoylchlorid (6.4 ml) tropfenweise zugegeben. Es entsteht eine fast klare Lösung aus der nach ca. 5min ein Niederschlag ausfällt. Man rührt noch ca. 2 h bei Raumtemperatur nach, gibt dann Methanol (100 ml) dazu und engt die Mischung im Vakuum ein. Der Rückstand wird mit Isopropanol verrührt, filtriert, mit wenig Methanol und Dieethylether gewaschen und im Vakuum getrocknet.
Ausbeute: 10.1 g
Fp.: <305°C
MS(DCI): 216 (M+H)⁺

### Beispiel 11

### N⁴-(Benzoyl)-N¹-methoxycarbonylmethylcytosin

N⁴-(Benzoyl)-cytosin (4.3 g) wird in trockenem DMF (60 ml) suspendiert und Natriumhydrid (0.48 g) in Portionen zugegeben. Die Mischung wird kurz auf 40°C erwärmt bis die Wasserstoffentwicklung beendet ist. Bei Raumtemperatur wird anschließend mit einer Spritze Bromessigsäuremethylester (2.1 ml) tropfenweise zugegeben. Man rührt noch 2.5 h bei Raumtemperatur nach und versetzt dann mit Methanol (10 ml). Das Lösungsmittel wird im Vakuum abgezogen und der verbliebene Rückstand mit Dichlormethan versetzt und dann filtriert. Der Filterrückstand wird noch mit wenig Wasser gewaschen und dann im Vakuum getrocknet.
Ausbeute: 6.45 g
Fp.: 234°C
MS(DCI): 288 (M+H)⁺
R_{f} = 0.85 (Ethylacetat:Methanol/8:2)

### Beispiel 12

### N⁴-(Benzoyl)-N¹-carboxymethylcytosin

N⁴-(Benzoyl)-N¹-methoxycarbonylmethylcytosin (5.85 g) wird in einer Mischung von Dioxan (80 ml) und Wasser (40 ml) gelöst und unter Rühren bei Raumtemperatur 2N wäßrige Natronlauge (11.25 ml) tropfenweise zugegeben (pH 11-12). Wenn die Verseifung des Methylesters beendet ist, -DC-Kontrolle- wird die Reaktionslösung mit 2M Kaliumhydrogensulfat-Lösung auf pH 3 gestellt. Der ausgefallene Niederschlag wird abgesaugt. Diese Rohprodukt wird in Natriumcarbonat-Lösung gelöst und erneut durch Zugabe von 2M Kaliumhydrogensulfat-Lösung ausgefällt. Das Produkt wird abgesaugt, mit wenig Wasser nachgewaschen und im Vakuum getrocknet.
Ausbeute: 5.84 g
Fp.: ab 283-286°C, Zers.
MS(ES⁺): 274 (M+H)⁺
R_{f} = 0.15 (Ethylacetat:Methanol/8:2)

### Beispiel 13

### N⁴-(tert.Butyloxycarbonyl)-cytosin

Cytosin (11.1 g) wird in trockenem Pyridin (250 ml) suspendiert. Dann werden Di-tert.butyldicarbonat (21.8 g) und 1 Spatelspitze 4-Dimethylaminopyridin zugegeben. Die Mischung wird 6 h bei 60°C gerührt wobei ein dicker Niederschlag entsteht. Die Reaktionslösung wird abgekühlt und der Niederschlag abgesaugt. Der Niederschlag wird in der Wärme mit Wasser verrührt, abgesaugt und im Vakuum getrocknet.
Ausbeute: 10.26 g
MS(DCI): 212 (M+H)⁺
R_{f} = 0.6 (Dichlormethan:Methanol/6:4)

### Beispiel 14

### N⁴-(tert.Butyloxycarbonyl)-N¹-methoxycarbonylmethylcytosin

N⁴-(tert.Butyloxycarbonyl)-cytosin (1.6 g) wird in trockenem DMF (30 ml) suspendiert, Natriumhydrid (0.19 g) in Portionen zugegeben und die Mischung 1.5 h bei Raumtemperatur gerührt bis die Wasserstoffentwicklung beendet ist. Bei Raumtemperatur wird anschließend mit einer Spritze Bromessigsäuremethylester (0.84 ml) tropfenweise zugegeben. Man rührt noch 5h bei Raumtemperatur nach und versetzt dann mit Methanol (1 ml). Das Lösungsmittel wird im Vakuum abgezogen und der verbliebene Rückstand mittels Säulenchromatographie an Kieselgel mit Dichlormethan als Elutionsmittel gereinigt. Die das Produkt enthaltenden Fraktionen werden vereinigt und im Vakuum eingeengt.
Ausbeute: 1.1 g
MS(DCI): 284 (M+H)⁺
R_{f} =0.5 (Dichlormethan:Methanol/95:5)

### Beispiel 15

### N⁴-(tert.Butyloxycarbonyl)-N¹-carboxymethylcytosin

N⁴-(tert.Butyloxycarbonyl)-N¹-methoxycarbonylmethylcytosin (4.2 g) wird in Wasser (30 ml) suspendiert und bei 0°C tropfenweise mit 2N wäßriger Natronlauge unter pH-Kontrolle (pH12) versetzt bis der Methylester verseift ist. Der Fortgang der Reaktion wird mittels DC verfolgt. Dann wird die Reaktionslösung mit Essigsäure auf pH 3 gestellt und das Lösungsmittel im Vakuum abdestilliert. Die Reinigung des Rohproduktes erfolgt mit Säulenchromatographie an Kieselgel mit Dichlormethan:Methanol:Triethylamin /8:1:1 als Elutionsmittel. Die das Produkt enthaltenden Fraktionen werden vereinigt und im Vakuum eingeengt.
Ausbeute: 3.5 g
Fp.: 95-98°C, Zers.
MS(FAB/NBA): 270.2 (M+H)⁺
R_{f} = 0.35 (Dichlormethan:Methanol:Triethylamin/8:1:1)

### Beispiel 16

### N⁶-(4-Methoxybenzoyl)-adenin

Adenin (13.5 g) wird in trockenem Pyridin (250 ml) suspendiert. Dann wird mit einer Spritze 4-Methoxybenzoylchlorid (17.06 g) tropfenweise zugegeben. Das Gemisch wird 3 h bei 100°C gerührt und über Nacht bei Raumtemperatur stehengelassen. Dann wird die Reaktionslösung mit Methanol versetzt und anschließend das Lösungsmittel im Vakuum abdestilliert. Man koevaporiert den Rückstand noch zweimal mit wenig Toluol und verrührt dann mit heißem Isopropanol. Man läßt die Mischung langsam abkühlen, saugt das ausgefallene Produkt ab und trocknet es im Vakuum.
Ausbeute: 22.2 g
Fp.: 212-214°C
MS(ES⁺): 270 (M+H)⁺
R_{f} = 0.67 (Dichlormethan:Isopropanol/8:2)

### Beispiel 17

### N⁶-(4-Methoxybenzoyl)-N⁹-methyloxycarbonylmethyladenin

N⁶-(4-Methoxybenzoyl)-adenin (8.01 g) wird in trockenem DMF (150 ml) suspendiert, Natriumhydrid (0.75 g) in Portionen zugegeben und die Mischung 0.5 h bei Raumtemperatur gerührt bis die Wasserstoffentwicklung beendet ist. Bei Raumtemperatur wird anschließend mit einer Spritze Bromessigsäuremethylester (2.85 ml) tropfenweise zugegeben. Man rührt noch 2 h bei Raumtemperatur nach und versetzt dann mit wenig Kohlendioxid in Methanol. Das Lösungsmittel wird im Vakuum abgezogen und der verbliebene Rückstand mit Dichlormethan/Wasser versetzt und dann filtriert. Der Filterrückstand wird noch mit wenig Wasser gewaschen und dann im Vakuum getrocknet.
Ausbeute: 4.03 g
MS(ES⁺): 342 (M+H)⁺
R_{f} = 0.76 (Ethylacetat:Methanol/8:2)

### Beispiel 18

### N⁶-(4-Methoxybenzoyl)-N⁹-carboxymethyladenin

N⁶-(4-Methoxybenzoyl)-N⁹-methyloxycarbonylmethyladenin (1.71 g) wird in Wasser (40 ml) suspendiert und bei 0°C tropfenweise mit 2N wäßriger Natronlauge unter pH-Kontrolle (pH 11.2) versetzt bis der Methylester verseift ist. Der Fortgang der Reaktion wird mittels DC verfolgt. Dann wird die Reaktionslösung filtriert und das Filtrat mit 2M Kaliumhydrogensulfat-Lösung auf pH 3 gestellt wobei das Produkt ausfällt. Man filtriert den Niederschlag ab, wäscht mit wenig kaltem Wasser nach und trocknet im Vakuum.
Ausbeute: 1.52 g
Fp.: 222-223°C, Zers.
MS(ES⁺): 328 (M+H)⁺
R_{f} = 0.2 (n-Butanol/Essigsäure/Wasser 3:1:1)

### Beispiel 19

### N²-(Isobutanoyl)-guanin

Guanin (3.02 g) wird in trockenem DMF (40 ml) suspendiert und mit Triethylamin (1.45 ml) versetzt. Dann wird mit einer Spritze Isobuttersäurechlorid (2.12 g) tropfenweise zugegeben. Das Gemisch wird 3 h bei 100°C gerührt, dann die Reaktionslösung mit Methanol versetzt und anschließend das Lösungsmittel im Vakuum abdestilliert. Man verrührt den Rückstand mit heißem Isopropanol, saugt das ausgefallene Produkt ab und trocknet es im Vakuum.
Ausbeute: 2.67 g
R_{f} = 0.45 (n-Butanol/Essigsäure/Wasser 3:1:1)

### Beispiel 20

### N²-(Isobutanoyl)-N⁹-methyloxycarbonylmethylguanin

N²-(Isobutanoyl)-guanin (4.42 g) wird in trockenem DMF (50 ml) suspendiert, Natriumhydrid (0.5 g) in Portionen zugegeben und die Mischung 1 h bei Raumtemperatur gerührt bis die Wasserstoffentwicklung beendet ist. Bei Raumtemperatur wird anschließend mit einer Spritze Bromessigsäuremethylester (1.9 ml) tropfenweise zugegeben. Man rührt noch 1 h bei Raumtemperatur nach und versetzt dann mit wenig Kohlendioxid in Methanol. Das Lösungsmittel wird im Vakuum abgezogen und der verbliebene Rückstand mittels Säulenchromatographie an Kieselgel mit Dichlormethan:Methanol/95:5 als Elutionsmittel gereinigt. Die das Produkt enthaltenden Fraktionen werden vereinigt und im Vakuum eingeengt.
Ausbeute: 2.35 g
MS(DCI): 294(M+H)⁺
R_{f} = 0.58 (Dichlormethan:Methanol/9:1)

### Beispiel 21

### N²-(Isobutanoyl)-N⁹-carboxymethylguanin

N²-(Isobutanoyl)-N⁹-methyloxycarbonylmethylguanin (9.68 g) wird in Wasser (100 ml) suspendiert und bei 0°C tropfenweise mit 2N wäßriger Natronlauge unter pH-Kontrolle (pH 11) versetzt bis der Methylester verseift ist. Der Fortgang der Reaktion wird mittels DC verfolgt. Dann wird die Reaktionslösung filtriert, das Filtrat mit 2M Kaliumhydrogensulfat-Lösung auf pH 3 gestellt und mit Ethylacetat extrahiert. Die organische Phase wird verworfen. Die wäßrige Phase wird im Vakuum auf ca. 20 ml Volumen konzentriert und mit Ethylacetat überschichtet. Der sich langsam abscheidende Niederschlag wird abgesaugt und getrocknet.
Ausbeute: 1.35 g
MS(ES⁺): 342(M+H)⁺
R_{f} = 0.34 (n-Butanol:Essigsäure:Wasser/3:1:1)

### Beispiel 22

### Herstellung von N-(4-Methoxyphenyl-diphenyl-methylaminoethyl)glycinmethylester Mmt-Aeg-OMe

14.76 g N-(Aminoethyl)glycinmethylester-Dihydrochlorid (H-Aeg-OMe · 2 HCl) werden in 300 ml DMF suspendiert und unter Rühren mit 30.2 ml Triethylamin versetzt. Man kühlt auf ca. 4°C und gibt unter gutem Rühren 22.23 g Mmt-Cl gelöst in 100 ml Dichlormethan langsam zutropfen. Man rührt noch 2.5 h bei Raumtemperatur nach. Dann filtriert man von ausgefallenem Triethylaminhydrochlorid ab, gibt 10 ml Methanol zur Lösung und engt das Filtrat im Vakuum ein. Der Rückstand wird dann an Kieselgel mit einer Mischung aus Diethylether/Petrolether/Triethylamin 200:100:3 chromatographiert. Die das Produkt enthaltenen Fraktionen werden vereinigt und im Vakuum zur Trockene eingedampft.
Ausbeute: 18.82 g Öl, das beim Stehenlassen langsam kristallisiert
R_{f} = 0.16 (Diethylether/Petrolether 2:1 mit 1 %Triethylamin)
MS(FAB/MeOH/NBA/LiCl): 411.2 (M+Li)⁺

### Beispiel 23

### Synthesevorschrift für die Herstellung von N-((4-Methoxyphenyl)-diphenylmethylamino)ethyl-N-((1-thyminyl)acetyl)glycin (Mmt-Aeg(T)-OH)

1.21 g (3 mMol) Mmt-Aeg-OMe werden in 20 ml THF gelöst und dann unter Kühlung und gutem Rühren 0.24 ml Chloracetylchlorid gelöst in 10 ml THF gleichzeitig mit 0.42 ml Triethylamin gelöst in 10 ml THF langsam zugetropft. Danach läßt man noch 30 min nachreagieren, saugt vom ausgefallenen Triethylaminhydrochlorid ab und versetzt die Lösung mit 30 ml trockenem DMF. Anschließend zieht man das THF im Vakuum am Rotationsverdampfer ab und gibt zu der Lösung von Mmt-Aeg(Chloracetyl)-OMe in DMF nacheinander 0.756 g (6 mMol) Thymin und 1.66 g (12 mMol) feingepulvertes Kaliumcarbonat. Diese Mischung läßt man 48 h bei Raumtemperatur rühren und saugt dann von dem Ungelösten ab. Das Lösungsmittel wird abgezogen und der Rückstand zwischen Wasser und Ethylacetat verteilt. Das in der Essigesterphase verbliebene Rohprodukt wird nach Abziehen des Lösungsmittels in einer Mischung von Dioxan/Methanol/Wasser durch Zugabe von 35 ml 0.2 N NaOH verseift. Der nach Abziehen des Lösungsmittels erhaltene Rückstand wird dann an Kieselgel mit Dichlormethan/MeOH/Triethylamin (100/10/1) chromatographisch gereinigt. Die das Produkt enthaltenen Fraktionen werden vereinigt und eingeengt. Es verbleiben 0.81 g eines farblosen Schaumes.
R_{f} = 0.29 (Dichlormethan/Methanol/Triethylamin 100:10:1)
MS(ES⁻): 1112.0 (2M-H)⁻, 555.3 (M-H)⁻

### Beispiel 24

### N-((4-Methoxyphenyl)-diphenylmethylamino)ethyl-N-((1-thyminyl)acetyl)glycinmethylester (Mmt-Aeg(T)-OMe)

1.00 g (2.48 mmol) N-((4-Methoxyphenyl)-diphenylmethyl)-aminoethylglycinmethylester werden in 5 ml DMF gelöst. Zu dieser Lösung gibt man 0.404 g (2.48 mmol) 3,4-Dihydro-3-hydroxy-4-oxo-1,2,3-benzotriazine und 0.632 ml (4.96 mmol) 4-Ethylmorpholin. Dann wird eine Lösung von 0.456 g (2.48 mmol) N-1-Carboxymethylthymine in 5 ml DMF hinzugetropft, gefolgt von 0.46 ml (3.0 mmol) N,N'-Diisopropylcarbodiimid. Die Reaktionsmischung wird 20 h bei Raumtemperatur gerührt. Dann wird das Lösungsmittel im Vakuum entfernt und der Rückstand in Ethylacetat gelöst. Diese Lösung wird zweimal mit Wasser und gesättigter Kaliumchloridlösung gewaschen. Die organische Phase wird über Natriumsulfat getrocknet, filtriert und dann zur Trockene eingedampft. Das so erhaltene Rohprodukt wird an Kieselgel, das mit einer Mischung aus Dichlormethan/Methanol/Triethylamin (100/1/1) äquilibriert wurde, mit einem Gradienten von 1-5% Methanol in Dichlormethan chromatographisch gereinigt. Die das Produkt enthaltenden Fraktionen werden vereinigt und im Vakuum eingeengt. Man erhält 1.28 g Produkt als farblosen Schaum.
MS(FAB): 571.2 (M+H)⁺
R_{f} = 0.28 (CH₂Cl₂:MeOH/95:5)

### Beispiel 25

### N-((4-Methoxyphenyl)-diphenylmethylamino)ethyl-N-((1-thyminyl)acetyl) glycin (Mmt-Aeg(T))-OH

Das Produkt aus der obigen Reaktion wird in einer Mischung aus 10 ml Dioxan und 2 ml Wasser gelöst. die Lösung wird auf 0°C gekühlt und tropfenweise 1 N Natronlauge zugegeben bis ein pH-Wert von 11 erreicht ist. Nach 2 h Reaktionszeit ist die Reaktion beendet und die Lösung wird durch vorsichtige Zugabe von 2 N KHSO₄-Lösung auf pH 5 gestellt Die Lösung wird dreimal mit Ethylacetat extrahiert, die vereinigten organischen Phasen über Natriumsulfat getrocknet und im Vakuum eingeengt. Das so erhaltene Rohprodukt wird an Kieselgel mit einem Gradienten von 5-10% Methanol und 1 % Triethylamin in Dichlormethan chromatographisch gereinigt. Die das Produkt enthaltenden Fraktionen werden vereinigt und im Vakuum eingeengt. Durch Koevaporation mit Pyridin und anschließend Toluol wird noch vorhandenes überschüssiges Triethylamin entfernt. Man erhält 1.065 g Produkt als farblosen Schaum.
MS(ES⁻) 1112.0 (2M-H)⁻, 555.3 (M-H)⁻
R_{f} = 0.28 (CH₂Cl₂:MeOH/8:2)

### Beispiel 26

### N-((4-Methoxyphenyl)-diphenylmethylamino)ethyl-N-((1-(N⁴-benzoyl)cytosyl)acetyl)glycinmethylester (Mmt-Aeg(C^{Bz})-OMe)

1.10 g N-((4-Methoxyphenyl)-diphenylmethyl)-aminoethylglycinmethylester (2.72mmol) werden in 5 ml DMF gelöst und 0.444 g (2.72 mmol) 3,4-Dihydro-3-hydroxy-4-oxo-1,2,3-benzotriazin sowie 0.94 ml 4-Ethylmorpholin (5.45 mmol) zugegeben. Eine Suspension von 0.744 g (2.72 mmol) N⁴-Benzoyl-N¹-carboxymethylcytosin in 20 ml DMF wird zugefügt, gefolgt von 0.51 ml (3.27 mmol) N,N'-Diisopropylcarbodiimid. Die Reaktionsmischung wird 20 h bei Raumtemperatur gerührt. Dann wird das Lösungsmittel im Vakuum abgezogen und der Rückstand in Ethylacetat gelöst. Diese Lösung wird zweimal mit Wasser und einmal mit gesättigter Kochsalzlösung gewaschen. Die organische Phase wird über Natriumsulfat getrocknet, filtriert und im Vakuum eingeengt. Das so erhaltene Rohprodukt wird an Kieselgel, das mit einer Mischung aus Dichlormethan/Methanol/Triethylamin (100/1/1) äquilibriert war, mit einem Gradienten von 1-2% Methanol in Dichlormethan chromatographisch gereinigt. Die das Produkt enthaltenden Fraktionen werden vereinigt und im Vakuum eingeengt. Man erhält 0.96g Produkt als gelblich-weißen Schaum.
MS(ES⁻) 658.6 (M-H)⁻
R_{f} = 0.37 (CH₂Cl₂:MeOH/95:5)

### Beispiel 27

### N-((4-Methoxyphenyl)-diphenylmethylamino)ethyl-N-((1-(N⁴-benzoyl)-cytosyl)acetyl)glycin (Mmt-Aeg(C^{Bz}))-OH

2.26 g (3.43 mMol) N-((4-Methoxyphenyl)-diphenylmethylamino)ethyl-N-((1-(N⁴-benzoyl)-cytosyl)acetyl)glycinmethylester werden in 50 ml Dioxan gelöst. Die Lösung wird auf 0°C gekühlt und 34.4 ml 1N Natronlauge tropfenweise zugegeben. Nach 10 min wird durch tropfenweise Zugabe von 1N KHSO₄ auf pH 5 gestellt, von ausgefallenen Salzen abfiltriert und mit wenig Dioxan nachgewaschen. Die vereinigten Filtrate werden im Vakuum eingedampft und der Rückstand zweimal mit Methanol und Dichlormethan koevaporiert. Das so erhaltene Rohprodukt wird an Kieselgel mit einem Gradienten von 5-10% Methanol und 1 % Triethylamin in Dichlormethan chromatographisch gereinigt.

Die das Produkt enthaltenden Fraktionen werden vereinigt und im Vakuum eingeengt. Durch Koevaporation mit Pyridin und anschließend Toluol wird noch vorhandenes überschüssiges Triethylamin entfernt. Man erhält 1.306 g Produkt als fast weißen Schaum.
MS(ES⁻) 644.3 (M-H)⁻
R_{f} = 0.68 (CH₂Cl₂:MeOH/8:2)

### Beispiel 28

### N-((4-Methoxyphenyl)-diphenylmethylamino)ethyl-N-((1-(N⁴-(4-tert-butylbenzoyl)-cytosyl)acetyl)glycinmethylester (Mmt-Aeg(C^{tBuBz})-OMe)

1.00g (2.48 mmol) N-((4-Methoxyphenyl)-diphenylmethyl)-aminoethylglycinmethylester werden in 5 ml DMF gelöst. Zu dieser Lösung werden nacheinander 0.403 g (2.48mmol) 3,4-Dihydro-3-hydroxy-4-oxo-1,2,3-benzotriazin und 0.63 ml (4.96 mmol) 4-Ethylmorpholin gegeben. Dann fügt man eine Suspension von 0.740 g (2.48 mmol) N⁴-(4-tert-Butylbenzoyl)-N¹-carboxymethyl-cytosin in 5 ml DMF gefolgt von 0.47 ml N,N'-Diisopropylcarbodiimid hinzu. Die Mischung wird 20 h bei Raumtemperatur gerührt und dann im Vakuum zur Trockene eingedampft. Der Rückstand wird in Ethylacetat gelöst, mit Wasser, gesättigter Natriumchloridlösung gewaschen, über Natriumsulfat getrocknet, filtriert und im Vakuum eingeengt. Das Rohprodukt wird mit Kieselgelgel-Säule, die mit einer Mischung aus Dichlormethan/Ethylacetat/Triethylamin (50/50/1) äquilibriert wurde und Dichlormethan/Ethylacetat (1/1) als Elutionsmittel gereinigt. Die vereinigten, das Produkt enthaltenden Fraktionen werden im Vakuum eingeengt. Man erhielt das Produkt als gelblich-weißen Schaaum in einer Ausbeute von 1.635 g.
MS (FAB/MeOH,NBA,LiCl): 722.5 (M+Li)⁺
R_{f} = 0.31 (CH₂Cl₂:Ethylacetat/1:1)

### Beispiel 29

### N-((4-Methoxyphenyl)-diphenylmethylamino)ethyl-N-((1-(N⁴-(4-tert-butylbenzoyl)-cytosyl)acetyl)glycin (Mmt-Aeg(C^{tBuBz})-OH)

1.63 g (2.28 mmol) N-((4-Methoxyphenyl)-diphenylmethylamino)ethyl-N-((1-(N⁴-(4-tert-butylbenzoyl)-cytosyl)acetyl) glycin-methylester werden in einer Mischung aus 10 ml Dioxan und 1 ml Wasser gelöst und bei 0°C unter Rühren tropfenweise mit 4.56 ml 1 N NaOH versetzt. Nach 2h wird durch tropfenweise Zugabe von 1N KHSO₄ der pH auf 5 gestellt, von ausgefallenen Salzen abfiltriert und mit wenig Dioxan nachgewaschen. Die vereinigten Filtrate werden im Vakuum eingedampft und der Rückstand zweimal mit Methanol und Dichlormethan koevaporiert. Das so erhaltene Rohprodukt wird an Kieselgel mit einem Gradienten von 2-10% Methanol und 1 % Triethylamin in Dichlormethan chromatographisch gereinigt. Die das Produkt enthaltenden Fraktionen werden vereinigt und im Vakuum eingeengt. Durch Koevaporation mit Pyridin und anschließend Toluol wird noch vorhandenes überschüssiges Triethylamin entfernt. Man erhält 0.831 g Produkt als fast weißen Schaum.
MS(ES⁻) 700.7 (M-H)⁻
R_{f} = 0.28 (CH₂Cl₂:MeOH/9:1), 0.63 (CH₂Cl₂:MeOH/7:3)

### Beispiel 30

### N-((4-Methoxyphenyl)-diphenylmethyloxy)ethyl-N-((1-thyminyl)acetyl)glycin (Mmt-Oeg(T)-OH)

0.5g (1.28 mmol) N-((4-Methoxyphenyl)-diphenylmethyloxy)ethylglycin werden in 10 ml DMF suspendiert und 0.47 ml (1.92 mMol) BSA werden tropfenweise zugegeben. Nacheinander werden dann 0.7 ml (5.1 mmol) Triethylamin und 0.26 g (1.28 mmol) Chlorcarboxymethylthymin zugefügt. Die Reaktionsmischung wird 4 h bei Raumtemperatur gerührt, dann weitere 65 mg (0.32 mmol) Chlorcarboxymethylthymin zugegeben und noch für 16 h gerührt. Danach zieht man das Lösungsmittel im Vakuum ab und reinigt das Rohprodukt an einer Kieselgelsäule mit einem Gradienten von 5-15% Methanol und 1 % Triethylamin in Dichlormethan. Die das Produkt enthaltenden Fraktionen werden vereinigt und im Vakuum eingeengt. Das erhaltene bräunliche Öl wird in wenig Dichlormethan gelöst und durch Zugabe von Diethylether das Produkt ausgefällt. Man erhält das Produkt als fast weißes Pulver.
Ausbeute: 0.219 g
MS(ES⁻) 556.3 (M-H)⁻
R_{f} = 0.54 (CH₂Cl₂:MeOH/8:2)

### Beispiel 31

### N-((4-Methoxyphenyl)-diphenylmethylamino)ethyl-N-((1-(N⁴-(4-methoxybenzoyl)-cytosyl)acetyl)glycinmethylester (Mmt-Aeg(C^{MeOBz})-OMe)

N-((4-methoxyphenyl)-diphenylmethyl)-aminoethylglycinmethylester (4.00 g; 9.9 mmol) werden in DMF (50 ml) gelöst. Zu dieser Lösung gibt man nacheinander 4-Ethylmorpholin (3.44 ml), 3,4-Dihydro-3-hydroxy-4-oxo-1,2,3-benzotriazin (1.62 g), N⁴-(4-methoxybenzoyl)-N¹-carboxymethylcytosin (3.00 g; 9.9 mmol) und N,N'-Diisopropylcarbodiimid (1.87 ml). Die Reaktionsmischung wird 48 h bei Raumtemperatur gerührt. Dann wird das Lösungsmittel im Vakuum abgedampft und der Rückstand in Ethylacetat aufgenommen. Diese Lösung wird zweimal mit Wasser und einmal mit gesättigter Kaliumchloridlösung extrahiert. Die organische Phase wird über Natriumsulfat getrocknet, filtriert und im Vakuum eingeengt. Die Reinigung erfolgt mittels Säulenchromatographie an Kieselgel, das vorher mit Dichlormethan enthaltend 1 % Triethylamin äquilibriert worden ist. Die Elution erfolgt mit einem Gradienten von 0-2% Methanol in Dichlormethan enthaltend 1 % Triethylamin. Die das Produkt enthaltenden Fraktionen werden vereinigt und im Vakuum auf kleines Volumen eingeengt.

Der hierbei ausfallende Dicyclohexylharnstoff wird abfiltriert und das Filtrat im Vakuum eingedampft. Man erhält die gewünschte Verbindung als hellgelben Schaum.
Ausbeute: 6.72 g
MS (FAB/MeOH,NBA,LiCl): 696.3 (M+Li)⁺
R_{f} = 0.59 (Dichlormethan:Methanol/9:1)

### Beispiel 32

### N-((4-Methoxyphenyl)-diphenylmethylamino)ethyl-N-((1-(N⁴-(4-methoxybenzoyl)-cytosyl)acetyl)glycin (Mmt-Aeg(C^{MeOBz})-OH)

N-((4-methoxyphenyl)-diphenylmethyl)-aminoethyl-N-((1-(N⁴-(4-methoxybenzoyl))-cytosyl)acetyl) glycinmethylester (6.60 g; 9.6 mmol) werden in einer Mischung aus Dioxan (50 ml) und Wasser (50 ml) gelöst und bei 0°C tropfenweise mit 1M wäßriger Natronlauge (15 ml) in zwei Teilen versetzt. Nach 2 h Reaktionszeit wird die Lösung durch Zugabe von Ionenaustauscher (Dowex AG 50WX4, Pyridinium-Form) neutralisiert. Der Ionenaustauscher wird abfiltriert und mit wäßrigem Dioxan und Methanol nachgewaschen. Die vereinigten Filtrate werden im Vakuum zur Trockene eingedampft und durch Säulenchromatographie an Kieselgel durch Elution mit 10% Methanol in Dichlormethan (mit 1 % Triethylamin) gereinigt. Man erhält das Produkt als gelblich-weißen Schaum. NachUmkristallisation aus Ethylacetat erhält man ein farbloses Pulver.
Ausbeute: 4.00 g
MS(ES⁻): 674.1(M-H)⁻
R_{f} = 0.39 (Dichlormethan:Methanol/9:1)

### Beispiel 33

### N-((4-methoxyphenyl)-diphenylmethylamino)ethyl-N-(9-(N²-(Isobutanoyl)-guanosyl)acetyl)glycinmethylester (Mmt-Aeg(G^{iBu})-OMe)

N-((4-methoxyphenyl)-diphenylmethyl)-aminoethylglycinmethylester (1.45 g; 3.59 mmol) werden in DMF (7 ml) gelöst. Zu dieser Lösung gibt man nacheinander 4-Ethylmorpholine (1.24 ml; 7.17 mmol), 3,4-Dihydro-3-hydroxy-4-oxo-1,2,3-benzotriazin (0.585 g; 3.59 mmol), N²-(Isobutanoyl)-N⁹-carboxymethylguanin (1.00 g; 3.59 mmol) und N,N'-Diisopropylcarbodiimid (0.67 ml; 4.31 mmol). Die Reaktionsmischung wird 48 h bei 4°C gerührt. Dann wird das Lösungsmittel im Vakuum abgedampft und der Rückstand in Ethylacetat aufgenommen. Diese Lösung wird zweimal mit Wasser und einmal mit gesättigter Kaliumchloridlösung extrahiert. Die organische Phase wird über Natriumsulfat getrocknet, filtriert und im Vakuum eingeengt. Der verbleibende gelbe Schaum wird in wenig Ethylacetat gelöst und mit Eis gekühlt um noch enthaltenen Dicyclohexylharnstoff auszufällen. Nach Filtration wird das Filtrat im Vakuum eingeengt. Die Reinigung erfolgt mittels Säulenchromatographie an Kieselgel, das vorher mit Dichlormethan:Ethylacetat 1/1 enthaltend 1% Triethylamin äquilibriert worden ist. Man eluiert mit Dichlormethan:Ethylacetat 1/1. Die das Produkt enthaltenden Fraktionen werden vereinigt und im Vakuum eingeengt. Man erhält die gewünschte Verbindung als hellgelben Schaum.
Ausbeute: 1.181 g
MS (FAB/MeOH,NBA,LiCl): 678.3 (M+2Li-H)⁺; 672.3 (M+Li)⁺
R_{f} = 0.13 (Dichlormethan:Methanol/95:5)

### Beispiel 34

### N-((4-methoxyphenyl)-diphenylmethylamino)ethyl-N-(9-(N²-(isobutanoyl)-guanosyl)acetyl)glycin (Mmt-Aeg(G^{iBu})-OH)

N-((4-methoxyphenyl)-diphenylmethylamino)ethyl-N-(9-(N²-(isobutanoyl)-guanosyl)acetyl)glycinmethylester (1.15 g; 1.72 mmol) werden in Dioxan (10 ml) gelöst und über einen Zeitraum von 2.5 h bei 0°C tropfenweise mit 1M wäßriger Natronlauge (10.32 ml) in 5 Teilen versetzt. Nach weiteren 2 h Reaktionszeit bei Raumtemperatur wird die Lösung durch tropfenweise Zugabe von 2M wäßriger Kaliumhydrogensulfatlösung auf pH5 gestellt. Die ausgefallenen Salze werden abfiltriert und mit wenig Dioxan nachgewaschen. Die vereinigten Filtrate werden im Vakuum zur Trockene eingedampft und der Rückstand je zweimal mit Ethanol sowie Dichlormethan:Methanol 1/1 koevaporiert. Die Reingung erfolgt durch Säulenchromatographie an Kieselgel durch Elution mit einem Gradienten von 10-20% Methanol in Dichlormethan (mit 1 % Triethylamin). Man erhält das Produkt als weißen Schaum.
Ausbeute: 1.229 g
MS (ES⁻): 650.3(M-H)⁻
R_{f} = 0.25 (Dichlormethan:Methanol/8:2)

### Beispiel 35

### N-((4-Methoxyphenyl)-diphenylmethylamino)ethyl-N-(9-(N⁶-(4-methoxybenzoyl)-adenosyl)acetyl)glycinmethylester (Mmt-Aeg(A^{MeOBz})-OMe)

N-((4-methoxyphenyl)-diphenylmethyl)-aminoethylglycinmethyl ester (1.24 g; 3.06 mmol) werden in DMF (7 ml) gelöst. Zu dieser Lösung gibt man nacheinander 4-Ethylmorpholin (1.06 ml; 6.12 mmol), 3,4-Dihydro-3-hydroxy-4-oxo-1,2,3-benzotriazin (0.498 g; 3.06 mmol), N⁶-(4-methoxybenzoyl)-N⁹-carboxymethyladenin (1.00 g; 3.06 mmol) and N,N'-Diisopropylcarbodiimid (0.58 ml; 3.67 mmol).
Die Reaktionsmischung wird 48 h bei 4°C gerührt. Dann wird das Lösungsmittel im Vakuum abgedampft und der Rückstand in Ethylacetat aufgenommen. Diese Lösung wird zweimal mit Wasser und einmal mit gesättigter Kaliumchloridlösung extrahiert. Die organische Phase wird über Natriumsulfat getrocknet, filtriert und im Vakuum eingeengt. Der verbleibende gelbe Schaum wird in wenig Ethylacetat gelöst und mit Eis gekühlt um noch enthaltenen Dicyclohexylharnstoff auszufällen. Dieser wird abfiltriert und das Filtrat im Vakuum eingeengt. Die Reinigung erfolgt mittels Säulenchromatographie an Kieselgel, das vorher mit Dichlormethan:Ethylacetat 1/1 enthaltend 1% Triethylamin äquilibriert worden ist. Die Elution erfolgt mit Dichlormethan:Ethylacetat 1/1. Die das Produkt enthaltenden Fraktionen werden vereinigt und im Vakuum eingeengt. Man erhält die gewünschte Verbindung als hellgelben Schaum.
Ausbeute: 1.752 g
MS (FAB/MeOH,NBA,LiCl): 720.3 (M+Li)⁺
R_{f} = 0.21 (Dichlormethan:Methanol/95:5)

### Beispiel 36

### N-((4-Methoxyphenyl)-diphenylmethylamino)ethyl-N-(9-(N⁶-(4-methoxybenzoyl)-adenosyl)acetyl)glycin (Mmt-Aeg(A^{MeOBz})-OH)

N-((4-methoxyphenyl)-diphenylmethylamino)ethyl-N-(9-(N⁶-(4-methoxybenzoyl)-adenosyl)acetyl) glycinmethyl ester (1.70 g; 2.38 mmol) werden in Dioxan (10 ml) gelöst und über einen Zeitraum von 2.5 h bei 0°C tropfenweise mit 1M wäßriger Natronlauge (10.32 ml) in 5 Teilen versetzt. Nach weiteren 2 h Reaktionszeit bei Raumtemperatur wird die Lösung durch tropfenweise Zugabe von 2M wäßriger Kaliumhydrogensulfatlösung auf pH 5 gestellt. Die ausgefallenen Salze werden abfiltriert und mit wenig Dioxan nachgewaschen. Die vereinigten Filtrate werden im Vakuum zur Trockene eingedampft und der Rückstand je zweimal mit Ethanol sowie Dichlormethan:Methanol 1/1 koevaporiert. Die Reingung erfolgt durch Säulenchromatographie an Kieselgel durch Elution mit einem Gradienten von 10-20% Methanol in Dichlormethan (mit 1 % Triethylamin). Man erhält das Produkt als weißen Schaum.
Ausbeute: 1.619 g
MS (ES⁻): 698.3(M-H)⁻
R_{f} = 0.10 (Dichlormethan:Methanol/8:2)

### Beispiel 37

### Thyminyl-essigsäure (Herstellverfahren wie bei L. Kosynkina, W. Wang und T. Chyau Liang, Tetrahedron Lett. 1994, 5173-5176 beschrieben)

37.8 g Thymin werden in einer Lösung von 64.5 g Kaliumhydroxid in 200 ml Wasser gelöst. Bei 40°C wird dann unter gutem Rühren eine Lösung von 62.5 g Bromessigsäure in 100 ml Wasser innerhalb von 70 min zugetropft. Man rührt noch 1 h bei 40°C nach, läßt auf 20°C abkühlen und stellt die Lösung durch Zugabe von konz. Salzsäure auf pH 5.5. Man läßt das Gemisch 1.5 h bei 0°C stehen, saugt das ausgefallene unreagierte Thymin ab und stellt dann das Filtrat mit konz. Salzsäure auf pH 2.0 wobei das Produkt ausfällt. Man läßt noch 1 h bei 0°C stehen, saugt dann den Niederschlag, der im Exsiccator getrocknet wird.
Ausbeute: 52.19 g
R_{f} = 0.23 (Ethylacetat/Methanol/Eisessig 75:20:5)
MS(DCI): 185(M+H)⁺

### Beispiel 38

### Thyminyl-essigsäuremethylester

31.5 g Thymin werden in 800 ml trockenem DMF suspendiert und 69.4 g feingepulvertes Kaliumcarbonat hinzugefügt. Man schüttelt 3 h bei Raumtemperatur und gibt dann 23.1 ml Bromessigsäuremethylester hinzu. Die Mischung wird unter Argon über Nacht geschüttelt, dann der Niederschlag abfiltriert, mit DMF gewaschen und das Filtrat im Vakuum am Rotationsverdampfer eingeengt. Der Rückstand wird in einer Mischung aus 240 ml Wasser und 19 ml 2N HCl in einer Reibschale gut verrieben, dann der Niederschlag abgesaugt und mit Wasser nachgewaschen. Das so erhaltene noch wasserfeuchte Rohprodukt wird aus 450 ml Methanol umkristallisiert.
Ausbeute: 25.5 g
R_{f =} 0.78 (Ethylacetat/Methanol/Eisessig 80:10:5)
MS(DCI): 199.1(M+H)⁺

### Beispiel 39

### N-((4-Methoxyphenyl)-diphenylmethylamino)ethyl-N-((1-thyminyl)acetyl)glycinmethylester Mmt-Aeg(T)-OMe

19.84 g Mmt-Aeg-OMe werden in 450 ml THF gelöst und dann unter Kühlung und gutem Rühren zuerst 6.82 ml Triethylamin zugefügt und dann 3.92 ml Chloracetylchlorid gelöst in 60 ml THF langsam zugetropft. Nach etwa 2/3 des Zutropfens der Chloracetylchlorid-Lösung werden weitere 3.41 ml Triethylamin zugegeben. Nach beendeter Zugabe von Chloracetylchlorid läßt man noch 1 h nachreagieren, saugt vom ausgefallenen Triethylaminhydrochlorid ab und versetzt die Lösung mit 400 ml trockenem DMF. Anschließend zieht man das THF im Vakuum am Rotationsverdamfer ab und gibt diese Lösung von Mmt-Aeg(Chloracetyl)-OMe in DMF zu einer schon über Nacht vorreagierten Mischung von 12.36 g Thymin und 27.16 g feingepulvertem Kaliumcarbonat in 200 ml trockenem DMF. Diese Mischung läßt man weitere 16 h bei Raumtemperatur rühren und saugt dann von dem Ungelösten ab. Das Filtrat wird am Rotationsverdampfer im Vakuum eingeengt und der Rückstand zwischen Wasser und Ethylacetat verteilt. Die organische Phase wird über Natriumsulfat getrocknet und dann nach Abfiltrieren des Trockenmittels im Vakuum am Rotationsverdampfer auf ein Volumen von ca. 80 ml eingeengt. Diese Lösung wird dann in eine Mischung aus 600 ml Petrolether und 200 ml Diethylether eingerührt wobei das Produkt ausfällt.
Ausbeute: 25 g
R_{f} = 0.34 (Dichlormethan/Methanol/Triethylamin 100:5:1)

### Beispiel 40

### N-((4-Methoxyphenyl)-diphenylmethylamino)ethyl-N-((1-thyminyl)acetyl)-glycin Mmt-Aeg(T)-OH

5.7 g Mmt-Aeg(T)-OMe werden in 25 ml Wasser suspendiert und nacheinander mit 13.9 ml Triethylamin und 12.5 ml Dioxan versetzt. Die entstandene klare Lösung läßt man 72 h bei Raumtemperatur stehen und destilliert dann am Rotationsverdampfer im Vakuum das Lösungsmittel ab. Es wird noch dreimal mit etwas Toluol nachdestilliert. Der Rückstand wird ine einer Mischung aus je 20 ml Dioxan und Ethylacetat aufgenommen, von etwas Ungelöstem abfiltriert und das Filtrat in 500 ml Ether, der 1% Triethylamin enthält, eingerührt. Das ausgefallene Produkt wird abgesaugt und im Exsiccator getrocknet.
Ausbeute 4.1 g
R_{f} = 0.28 (Dichlormethan/Methanol/Triethylamin 100:10:1)

### Beispiel 41

### N-((4-Methoxyphenyl)-diphenylmethylamino)ethyl-N-((1-thyminyl)acetyl)-glycin Mmt-Aeg(T)-OH

5.7 g Mmt-Aeg(T)-OMe werden in einer Mischung aus 80 ml Wasser, 20 ml Methanol und 13.9 ml Triethylamin ge löst. Die entstandene klare Lösung läßt man 24 h bei Raumtemperatur rühren und destilliert dann am Rotationsverdampfer im Vakuum das Lösungsmittel ab. Es wird noch dreimal mit etwas Toluol nachdestilliert. Der Rückstand wird in einer Mischung aus je 20 ml Dioxan und Ethylacetat aufgenommen, von etwas Ungelöstem abfiltriert und das Filtrat in 500 ml Ether, der 1% Triethylamin enthält, eingerührt. Das ausgefallene Produkt wird abgesaugt und im Exsiccator getrocknet.
Ausbeute 4.8 g
R_{f} = 0.28 (Dichlormethan/Methanol/Triethylamin 100:10:1)

### Beispiel 42

### N-((2-Hydroxy)ethyl-N-((1-thyminyl)acetyl)glycin H-Oeg(T)-OH

2.76 g Thyminylessigsäure werden in 15 ml trockenem DMF gelöst und 4.92 g TOTU und 2.08 ml Triethylamin zugegeben. Die Mischung wird 30 min bei Raumtemperatur nachgerührt und dann langsam zu einer Lösung bestehend aus 3.57 g (2-Hydroxyethyl)-glycin, 10 ml Wasser, 10 ml DMF und 4.16 ml Triethylamin zugetropft. Man rührt noch 1 h bei Raumtemperatur nach und gibt dann 20 ml Acetonitril hinzu, wobei der Überschuß Hydroxyethylglycin ausfällt. Man saugt den Niederschlag ab und engt das Filtrat am Rotationsverdampfer im Vakuum ein. Der Rückstand wird in Wasser aufgenommen, mit 1 N Salzsäure auf pH 1.5 gestellt und mit Ethylacetat extrahiert. Die wäßrige Phase wird mit gesättigter Natriumhydrogencarbonatlösung auf pH 5 gestellt und am Rotationsverdampfer eingeengt. Der Rückstand wird mit 250 ml Ethanol versetzt und das dabei ausgefallenen Natriumchlorid abgesaugt. Das Filtrat wird eingeengt und das Rohprodukt an Kieselgel mit Dichlormethan/Methanol/Ethylacetat 10:2:1 unter Zusatz von 1% Triethylamin chromatographisch gereinigt. Die das Produkt enthaltenden Fraktionen werden vereinigt und am Rotationsverdampfer im Vakuum eingeengt.
Ausbeute: 2.39 g
R_{f} = 0.17 (Dichlormethan/Methanol/ Ethylacetat 10:2:1 + 1% Triethylamin)
MS(DCI): 286(M+H)⁺

### Beispiel 43

### N-(2-(Di-(4-Methoxyphenyl)-phenyl)methyloxy)ethyl-N-((1-thyminyl)acetyl)glycin Dmt-Oeg(T)-OH

1.76 g H-Oeg(T)-OH werden in 30 ml DMF gelöst, 3.41 ml Triethylamin hinzugegeben und bei 0°C eine Lösung von 4.16 g Dmt-Cl in 30 ml Dichlormethan innerhalb von 20 min zugetropft. Man rührt noch 3 h bei Raumtemperatur nach, filtriert dann das ausgefallene Triethylaminhydrochlorid ab und engt das Filtrat am Rotationsverdampfer im Vakuum ein. Die Reinigung des Rohproduktes erfolgt an Kieselgel mit Dichlormethan/Methanol/Ethylacetat 10:2:1 unter Zusatz von 1% Triethylamin. Die das Produkt enthaltenden Fraktionen werden vereinigt und am Rotationsverdampfer im Vakuum eingeengt.
Ausbeute: 3.2 g
R_{f} = 0.29 (Dichlormethan/Methanol/ Ethylacetat 10:2:1 + 1% Triethylamin)
MS(ES⁺ + LiCl): 594.3(M+Li)+, 600.4(M+2Li-H)⁺

### Beispiel 44

### Herstellung von N-(2-(Di-(4-Methoxyphenyl)-phenyl)methylthio)ethyl-N-((1-thyminyl)acetyl)glycin Dmt-Teg(T)-OH

### Beispiel 44a: Dmt-S-CH₂-CH₂-NH₂

10.17 g Dmt-Cl werden in 100 ml Essigsäure gelöst und 4.43 g 2-Mercaptoethylamin-Hydrochlorid gelöst in 70 ml Wasser hinzugefügt. Man rührt noch 2h bei Raumtemperatur nach und engt dann am Rotationsverdampfer das Gemisch auf ein Volumen von ca. 50 ml ein. Dann werden 200 ml Wasser hinzugegeben und die Lösung mit 2N NaOH auf pH 10 gestellt, wobei die Substanz ausfällt. Die Mischung wird mit 3mal 100 ml Ethylacetat extrahiert, die vereinigten organischen Phasen mit ges. NaCl-Lösung gewaschen und über Natriumsulfat getrocknet. Das Trockenmittel wird abfiltriert und das Filtrat am Rotationsverdampfer im Vakuum eingeengt. Das so erhaltene Rohprodukt wird ohne weitere Reinigung in die nächste Reaktion eingesetzt.
Ausbeute: 14.75 g

### Beispiel 44b: Dmt-S-CH₂-CH₂-NH-CH₂-CO₂C₂H₅

12.56 g Dmt-S-CH₂-CH₂-NH₂ (Rohprodukt aus 44a) werden in 100 ml trockenem DMF gelöst und unter Rühren bei Raumtemperatur nacheinander mit 4.6 ml Triethylamin und 3.66 ml Bromessigsäureethylester versetzt. Man rührt noch weitere 3h nach, filtriert von etwas Niederschlag ab und engt das Filtrat im Vakuum am Rotationsverdampfer ein. Der Rückstand wird in 150 ml Ethylacetat aufgenommen, 4mal mit je 40 ml Wasser gewaschen, die organische Phase über Natriumsulfat getrocknet und nach Abfiltrieren des Trockenmittels das Filtrat im Vakuum am Rotationsverdampfer eingeengt. Das so erhaltene Rohprodukt wird ohne weitere Reinigung in die nächste Reaktion eingesetzt.
Ausbeute: 12.92 g

### Beispiel 44c: Dmt-Teg(T)-OEt

5.95 g Dmt-S-CH₂-CH₂-NH-CH₂-CO₂C₂H₅ werden in 50 ml trockenem DMF gelöst und nacheinander mit 2.36 g Thyminylessigsäure, 4.45 ml Triethylamin und 4.2 g TOTU versetzt. Man rührt die Mischung 2h bei Raumtemperatur und läßt über Nacht stehen. Dann engt man das Reaktionsgemisch im Vakuum am Rotationsverdampfer ein, nimmt den Rückstand in 150 ml Ethylacetat auf, wäscht mit 5mal je 10 ml ges. Natriumhydrogencarbonat-Lösung und zweimal 10 ml Wasser und trocknet die organische Phase über Natriumsulfat. Das Trockenmittel wird abfiltriert und das Filtrat am Rotationsverdampfer im Vakuum eingeengt. Man erhält ein öliges Rohprodukt das ohne weitere Reinigung in die nächste Reaktion eingesetzt wird.
Ausbeute: 8.06 g

### Beispiel 44d: Dmt-Teg(T)-OH

8.06 g Dmt-Teg(T)-OEt werden in einer Mischung aus 80 ml Dioxan und 40 ml Wasser gelöst und durch portionsweise Zugabe von insgesamt 13 ml 2 N Natronlauge verseift. Dann wird die Lösung am Rotationsverdampfer im Vakuum auf ca. 50 ml eingeengt und mit viermal 50 ml Ethylacetat extrahiert. Dann wird die wäßrige Phase mit 2N Salzsäure auf pH 5 gestellt und viermal 50 ml Ethylacetat extrahiert. Die vereinigten organischen Phasen werden mit zweimal 30 ml Wasser gewaschen und über Natriumsulfat getrocknet. Das Trockenmittel wird abfiltriert und das Filtrat am Rotationsverdampfer im Vakuum auf ca. 30 ml Volumen eingeengt. Man gibt etwas Triethylamin zu und rührt die Lösung in 300 ml Diisopropylether ein wobei das Produkt ausfällt. Zur weiteren Reinigung wird an Kieselgel mit einem Stufengradienten von 0-5% Methanol in Dichlormethan (1% Triethylamin in allen Elutionsmitteln) chromatographiert. Die das Produkt enthaltenden Fraktionen werden vereinigt und am Rotationsverdampfer im Vakuum eingeengt.
Ausbeute: 2.38 g
R_{f} = 0.38 (Dichlormethan/Methanol/Triethylamin 100:10:1)
MS(FAB/NBA + LiCl): 610.2(M+Li)⁺, 616.2(M+2Li-H)⁺

### Beispiel 45

### 2-N-Acetyl-4-O-diphenylcarbamoyl-9-methoxycarbonylmethyl-guanin

15.52 g 2-N-Acetyl-4-O-diphenylcarbamoyl-guanin (hergestellt wie bei R. Zou und M.J. Robins, Can J. Chem. 65,1436-1437,1987 beschrieben) werden in 200 ml trockenem DMF suspendiert und nach Zugabe von 13.6 ml Diisopropylethylamin kurz erwärmt bis eine klare Lösung entstanden ist. Dann werden 4.04 ml Bromessigsäuremethylester zugegeben und die Lösung über Nacht gerührt. Anschließend wird das Lösungsmittel im Vakuum am Rotationsverdampfer entfernt und der Rückstand in 200 ml Methanol gelöst. Diese Lösung wird unter kräftigem Rühren in 600 ml Wasser eingetropft, wobei das Produkt ausfällt. Das so erhaltene Rohprodukt wird abgesaugt, mit Wasser gewaschen, erneut in Methanol gelöst und nach Einengen zur Trockene mit Ethylacetat verrieben. Das erhaltene Produkt wird abgesaugt, mit Ethylacetat und Ether gewaschen und dann im Vakuum getrocknet. Aus der Mutterlauge erhält man nach Einengen und erneutes Verreiben mit Ethylacetat weiteres Produkt.
Ausbeute: 13.2 g
MS(ES⁺): 461.3(M+H)⁺
R_{f} = 0.79 (2-Butanon/Pyridin/Wasser/Essigsäure 70:15:15:2)

### Beispiel 46

### 2-N-Acetyl-4-O-diphenylcarbamoyl-9-carboxymethyl-guanin

7.54 g 2-N-Acetyl-4-O-diphenylcarbamoyl-9-methoxycarbonylmethyl-guanin werden in einer Mischung aus 20 ml Methanol, 80 ml Dioxan und 40 ml Wasser gelöst und bei pH 13 mit insgesamt 18 ml 1N NaOH verseift. Dann wird die Lösung durch Zugabe von 1N Salzsäure auf pH 6 gestellt und im Vakuum am Rotationsverdampfer auf ein Volumen von ca. 50 ml eingedampff. Nach Zugabe von 400 ml Wasser wird mit 1N Salzsäure auf pH 3 gestellt wobei das Produkt ausfällt. Das Produkt wird abgesaugt, mit Wasser gewaschen und im Exsiccator getrocknet.
Ausbeute: 5.92 g
MS(ES⁺): 447.2(M+H)⁺
R_{f} = 0.48 (2-Butanon/Pyridin/Wasser/Essigsäure 70:15:15:2)

### Beispiel 47

### N-((4-Methoxyphenyl)-diphenylmethylamino)ethyl-N-((9-(N²-acetyl-O⁴-diphenylcarbamoyl)guanosyl)acetyl)glycinmethylester Mmt-Aeg(G^{2-Ac,4-Dpc})-OMe

3.57 g 2-N-Acetyl-4-O-diphenylcarbamoyl-9-carboxymethyl-guanin werden in 120 ml trockenem DMF gelöst und nacheinander 3.23 g Mmt-Aeg-OMe, 2.62 g TOTU und in Portionen 4.08 ml Diisopropylethylamin zugegeben. Die Mischung wird 4 h bei Raumtemperatur geührt und über Nacht stehen gelassen. Dann wird das Lösungsmittel im Vakuum am Rotationsverdampfer abdestilliert und der Rückstand zwischen 160 ml Ethylacetat und 20 ml Wasser verteilt. Die organische Phase wird dreimal mit je 20 ml Wasser extrahiert, über Natriumsulfat getrocknet, das Trockenmittel abfiltriert und das Filtrat eingeengt. Der verbliebene Rückstand wird mehrmals mit Diethylether verrieben, das dann als Niederschlag ausgefallene Produkt abgesaugt und im Exsiccator getrocknet.
Ausbeute: 5.53 g
R_{f} = 0.63 (Dichlormethan/Methanol/Triethylamin 100:5:1)

### Beispiel 48

### N-((4-Methoxyphenyl)-diphenylmethylamino)ethyl-N-((9-(N²-acetyl-O⁴-diphenylcarbamoyl)guanosyl)acetyl)glycin Mmt-Aeg(G^{2-Ac,4-Dpc})-OH

4.99 g Mmt-Aeg(G^{2-Ac,4-Dpc})-OMe werden in einer Mischung aus 50 ml Dioxan und 10 ml Wasser gelöst und durch portionsweise Zugabe von insgesamt 15 ml 0.5N NaOH verseift. Dann wird die Lösung mit 0.5N HCl auf pH 9 gestellt und das Lösungsmittel im Vakuum am Rotationsverdampfer abgezogen. Man destilliert noch einmal mit etwas Toluol nach und löst dann den Rückstand in 30 ml Methanol. Diese Lösung wird in 300 ml Diethylether (mit 1% Triethylamin versetzt) eingerührt wobei das Produkt ausfällt. Der noch etwas klebrige Niederschlag wird mit Diethylether verrieben, abgesaugt und im Exsiccator getrocknet. Die weitere Reinigung erfolgt mittels Chromatographie an Kieselgel mit einem Stufengradienten (4-16 %) von Methanol/Ethylacetat (1:1) in Dichlormethan mit 1% Triethylamin in allen Lösungsmitteln. Die das Produkt enthaltenden Fraktionen werden vereinigt und im Vakuum am Rotationsverdampfer eingeengt.
Ausbeute: 2.68 g
R_{f} = 0.18 (Dichlormethan/Methanol/Triethylamin 100:7.5:1)
MS(ES⁺ + LiCl): 819.4(M+H)⁺, 825.3(M+Li)⁺

### Beispiel 49

### 1-Acetyl-2,4-dihydroxy-5-methyl-pyrimidin 1-Acetyl-thymin

75 g Thymin werden in 375 ml Acetanhydrid suspendiert und 45 min unter Rückfluß erhitzt. Danach wird die Mischung auf 0°C gekühlt, wobei das Produkt ausfällt. Der Niederschlag wird abgesaugt und mit 375 ml Ethylacetat verrührt. Man saugt den Niederschlag ab, wäscht mit etwas Diethylether nach und trocknet.
Ausbeute: 89.1 g
Schmelzpunkt: 187-189°C
R_{f} = 0.67 (Dichlormethan/Methanol 95:5)

### Beispiel 50

### 3-Benzyloxymethyl-2,4-dihydroxy-5-methyl-pyrimidin 3-Benzyloxymethyl-thymin

52.0 g 1-Acetyl-2,4-dihydroxy-5-methyl-pyrimidin werden in 300 ml DMF suspendiert und nach Zugabe von 47.5 ml Triethylamin auf 0°C gekühlt. Zur gut gerührten Mischung werden langsam 100 ml Benzylchlormethylether in 50 ml DMF bei dieser Temperatur zugetropft. Man rührt noch 1 h nach und läßt über Nacht bei Raumtemperatur stehen. Dann wird die Reaktionsmischung im Vakuum am Rotationsverdampfer eingeengt, in 450 ml Methanol und 300 ml einer 40%igen wäßrigen Lösung von Methylamin aufgenommen und 1.5 h am Rückfluß gekocht. Die Lösung wird dann eingeengt und mit 10.8 g Natriumhydrogencarbonat in 600 ml Wasser verrührt. Das ausgefallene Produkt wird abgesaugt, mit Wasser und Ethanol gewaschen und aus Ethanol umkristallisiert.
Ausbeute: 46.66 g
Schmelzpunkt: 119-120°C
R_{f} = 0.38 (Dichlormethan/Methanol 95:5)

### Beispiel 51

### 3-Benzyloxymethyl-2,4-dihydroxy-5-methyl-pyrimidin-essigsäureethylester 3-Benzyloxymethyl-1-ethoxycarbonylmethyl-thymin

4.97g Natriumhydrid werden in 150 ml THF vorgelegt, dann 45.04 g 3-Benzyloxymethyl-2,4-dihydroxy-5-methyl-pyrimidin gelöst in 550 ml THF unter N₂-Atmosphäre bei 0°C zugetropft und anschließend 21.42 ml Bromessigsäureethylester in 700 ml THF bei dieser Temperatur zugegeben. Danach läßt man noch 5h bei Raumtemperatur nachrühren und gibt anschließend vorsichtig Wasser hinzu. Die organische Phase wird abgetrennt und die Wasserphase noch viermal mit Dichlormethan extrahiert. Die vereinigten organischen Phasen werden über Magnesiumsulfat getrocknet, eingeengt und der Rückstand mit Heptan verrührt.
Ausbeute: 52.4 g
R_{f} = 0.15 (Dichlormethan/Methanol 95:5)
MS(FAB, NBA): 319.1(M+H)⁺

### Beispiel 52

### 2,4-Dihydroxy-5-methyl-pyrimidin-essigsäureethylester Thyminylessigsäureethylester

25 g 3-Benzyloxymethyl-2.4-dihydroxy-5-methyl-pyrimidin-essigsäureethylester werden in 1000 ml Dichlormethan gelöst, die Mischung auf -70°C abgekühlt und bei dieser Temperatur unter gutem Rühren 375 ml einer 1M Lösung von Bortrichlorid in Dichlormethan zugetropft. Man läßt noch 3 h bei dieser Temperatur nachreagieren und tropft dann bei -70° bis -60°C 320 ml Methanol zu. Nach 1 h gibt man 197.05 ml Triethylamin hinzu und läßt die Mischung auf Raumtemperatur kommen. Dann gibt man noch etwas Wasser hinzu und engt das Gemisch am Rotationsverdampfer ein. Der Rückstand wird in Ethylacetat aufgenommen und mit Wasser gewaschen. Die organische Phase wird über Magnesiumsulfat getrocknet und dann zur Trockene eingeengt.
Ausbeute: 12 g
R_{f} = 0.45 (Dichlormethan/Methanol 9:1)
MS(DCI): 199(M+H)⁺

### Beispiel 53

### 2,4-Dihydroxy-5-methyl-pyrimidin-essigsäure Thyminylessigsäure

10 g 2,4-Dihydroxy-5-methyl-pyrimidin-essigsäureethylester werden in 130 ml Dioxan/Wasser (1:1) gelöst und dann nach Zugabe von 60 ml 1N LiOH über Nacht bei Raumtemperatur gerührt. Dann wird die Mischung auf kleines Volumen eingeengt, die wäßrige Phase mit Ether gewaschen und dann auf pH 2.5 gestellt. Hierbei fällt das Produkt aus. Man saugt ab und erhält 4.9 g Produkt. Aus der Mutterlauge lassen sich durch extrahieren mit Pentanol und ausfällen mit Heptan/Ether weitere 1.5 g Produkt erhalten.
Ausbeute: 6.4 g
R_{f} = 0.30 (Dichlormethan/Methanol 3:2)
MS(DCI): 185(M+H)⁺

### Beispiel 54

### N-1 Chlorocarboxymethylthymin Thyminylessigsäurechlorid

### Beispiel 54a:

2 g 2,4-Dihydroxy-5-methyl-pyrimidin-essigsäure werden 3h mit 15 ml Thionylchlorid bei 60°C gerührt bis keine Gasentwicklung mehr auftritt. Dann wird das überschüssige Thionylchlorid am Rotationsverdampfer im Vakuum abgezogeri und noch dreimal mit wenig Toluol nachdestilliert. Das so erhaltene Produkt wird direkt in die nachfolgende Reaktion eingesetzt.
Ausbeute: 2.4 g
MS(DCI): 203(M+H)⁺

### Beispiel 54b:

N-1 Carboxymethylthymin (3.0 g; 16.3 mmol) werden in Thionylchlorid (90 ml) suspendiert. Die Suspension wird für 1.5 h auf 70°C erhitzt und dann 16 h bei Raumtemperatur stehengelassen. Das überschüssige Thionylchlorid wird in Vakuum entfernt und der Rückstand dreimal mit Toluol koevaporiert. Das erhaltene Produkt wird zweimal mit n-Hexan verrieben und im Vakuum getrocknet. Man erhält die Verbindung als hell-oranges Pulver.
Ausbeute: 3.30 g
MS(DCI; Dichlormethan): 203 (M+H)⁺
R_{f} = 0.10 (Dichlormethan/Methanol 7:3).

### Beispiel 55

### Herstellung von N-((4-Methoxyphenyl)-diphenylmethylamino)ethyl-N-((1-imidazolyl)acetyl)glycinmethylester Mmt-Aeg(Im)-OMe

4.68 g Mmt-Aeg-OMe werden in 150 ml THF gelöst und dann unter Kühlung und gutem Rühren 0.95 ml Chloracetylchlorid gelöst in 15 ml THF gleichzeitig mit 3.3 ml Triethylamin gelöst in 10 ml THF langsam zugetropft. Danach läßt man noch 45 min nachreagieren, saugt vom ausgefallenen Triethylaminhydrochlorid ab und versetzt die Lösung mit 150 ml trockenem DMF. Anschließend zieht man das THF im Vakuum am Rotationsverdamfer ab und gibt zu der Lösung von Mmt-Aeg(Chloracetyl)-OMe in DMF nacheinander 1.62 g Imidazol und 6.55 g feingepulvertes Kaliumcarbonat. Nach 16 h saugt man von ungelöstem Salz ab und gibt zum Filtrat erneut 1.62 g Imidazol und 6.55 g feingepulvertes Kaliumcarbonat. Diese Mischung läßt man weitere 16 h bei Raumtemperatur rühren und saugt dann von dem Ungelösten ab. Das Filtrat wird am Rotationsverdampfer im Vakuum eingeengt und der Rückstand zwischen Wasser und Ethylacetat verteilt. Die organische Phase wird über Natriumsulfat getrocknet und dann nach Abfiltrieren des Trockenmittels eingedampft. Der Rückstand wird in Dichlormethan aufgenommen und durch Einrühren in eine Mischung aus Diethylether/Petrolether 4:1 ausgefällt. Der noch etwas klebrige Niederschlag wird nach Abdekantieren des Lösungsmittels und verreiben mit Ether fest.
Ausbeute: 3.96 g
R_{f} = 0.39 (Dichlormethan/Methanol/Triethylamin 100:5:1)

### Beispiel 56

### Herstellung von N-((4-Methoxyphenyl)-diphenylmethylamino)ethyl-N-((1-imidazolyl)acetyl)glycin Mmt-Aeg(Im)-OH

1.79 g N-((4-Methoxyphenyl)-diphenylmethylamino)ethyl-N-((1-imidazolyl)acetyl)-glycinmethylester werden in einer Mischung aus 50 ml Dioxan und 25 ml Wasser gelöst und durch Zugabe von 5.25 ml 1 N NaOH verseift. Dann wird die Lösung mit 1 N HCl auf pH 8 gestellt und im Vakuum am Rotationsverdampfer zur Trockene eingeengt. Der Rückstand wird noch zweimal mit wenig Toluol nachdestilliert und dann an Kieselgel mit Dichlormethan/Methanol/Ethylacetat 100:10:5 mit 1% Triethylamin chromatographisch gereinigt. Die das Produkt enthaltenden Fraktionen werden vereinigt und m Vakuum am Rotationsverdampfer zur Trockene eingeengt.
Ausbeute: 1.70 g
R_{f} = 0.27 (Dichlormethan/Methanol/Triethylamin 100:20:1)
MS(ES⁺): 499.3 (M+H)⁺
Nachfolgend sind weitere Synthesen mit Chloracetylzwischenstufe beschrieben:

### Beispiel 57

### Herstellung der DMF-Lösung von N-((4-Methoxyphenyl)diphenylmethylamino)ethyl-N-(chloroacetyl)glycinmethylester Mmt-Aeg(Chloracetyl)-OMe

34.85 g Mmt-Aeg-OMe werden in 900 ml THF gelöst und dann unter Kühlung und gutem Rühren 6.9 ml Chloracetylchlorid gelöst in 110 ml THF gleichzeitig mit 24 ml Triethylamin gelöst in 110 ml THF langsam zugetropft. Danach läßt man noch 1 h nachreagieren, saugt vom ausgefallenen Triethylaminhydrochlorid ab und versetzt die Lösung mit 600 ml trockenem DMF. Anschließend zieht man das THF im Vakuum am Rotationsverdamfer ab. Die erhaltene Lösung von Mmt-Aeg(Chloracetyl)-OMe in DMF wird geteilt.

### Beispiel 58

### Herstellung von N-((4-Methoxyphenyl)-diphenylmethylamino)ethyl-N-((1-(4-Nitro)imidazolyl)acetyl)glycinmethylester Mmt-Aeg(Im^{4-Nitro})-OMe

Zu der einen Hälfte der oben hergestellten Lösung von Mmt-Aeg(Chloracetyl)-OMe in DMF gibt man 9.75 g feingepulvertes 4-Nitroimidazol und 23.85 g feingepulvertes Kaliumcarbonat. Diese Mischung läßt man weitere 16 h bei Raumtemperatur rühren und saugt dann von dem Ungelösten ab. Das Filtrat wird am Rotationsverdampfer im Vakuum eingeengt und der Rückstand zwischen Wasser und Ethylacetat verteilt. Die organische Phase wird dreimal mit je 50 ml Wasser gewaschen, über Natriumsulfat getrocknet und dann nach Abfiltrieren des Trockenmittels im Vakuum am Rotationsverdampfer auf ein Volumen von ca. 60 ml eingeengt. Diese Lösung wird dann in eine Mischung aus 400 ml Petrolether und 200 ml Diethylether eingerührt wobei das Produkt ausfällt. Das Produkt wird abgesaugt und im Exsiccator getrocknet.
Ausbeute: 19.8 g
R_{f} = 0.40 (Dichlormethan/Methanol/Triethylamin 100:5:1)
MS(ES⁺): 558.3 (M+H)⁺

### Beispiel 59

### Herstellung von N-((4-Methoxyphenyl)-diphenylmethylamino)ethyl-N-((1-(4-Nitro)imidazolyl)acetyl)glycin Mmt-Aeg(Im^{4-Nitro})-OH

5.37 g N-((4-Methoxyphenyl)-diphenylmethylamino)ethyl-N-((1-(4-Nitro)imidazolyl)acetyl)glycinmethylester werden in einer Mischung aus 20 ml Methanol, 80 ml Wasser und 13.9 ml Triethylamin gelöst. Die klare Lösung läßt man 48 h bei Raumtemperatur stehen. Danach ist das Ausgangsmaterial verseift und die Lösung wird im Vakuum am Rotationsverdampfer zur Trockene eingeengt. Der Rückstand wird noch zweimal mit wenig Toluol nachdestilliert Der Rückstand wird in einer Mischung aus je 20 ml Dioxan und Ethylacetat aufgenommen, von etwas Ungelöstem abfiltriert und das Filtrat in 500 ml Ether, der 1% Triethylamin enthält, eingerührt. Das ausgefallene Produkt wird abgesaugt mit etwas Ether gewaschen und im Exsiccator getrocknet.
Ausbeute: 4.21 g
R_{f} = 0.24 (Dichlormethan/Methanol/Triethylamin 100:10:1)
MS(ES⁺): 543.2 (M⁺)

### Beispiel 60

### Herstellung von N-((4-Methoxyphenyl)-diphenylmethylamino)ethyl-N-((1-(1,2,4)triazolyl)acetyl)glycinmethylester Mmt-Aeg(Triaz)-OMe

Zu der zweiten Hälfte der oben hergestellten Lösung von von Mmt-Aeg(Chloracetyl)-OMe in DMF gibt man 5.96 g feingepulvertes 1,2,4-Triazol und 23.85 g feingepulvertes Kaliumcarbonat. Diese Mischung läßt man weitere 16 h bei Raumtemperatur rühren und saugt dann von dem Ungelösten ab. Das Filtrat wird am Rotationsverdampfer im Vakuum eingeengt und der Rückstand zwischen Wasser und Ethylacetat verteilt. Die organische Phase wird dreimal mit je 50 ml Wasser gewaschen, über Natriumsulfat getrocknet und dann nach Abfiltrieren des Trockenmittels im Vakuum am Rotationsverdampfer auf ein Volumen von ca. 60 ml eingeengt. Diese Lösung wird dann in eine Mischung aus 400 ml Petrolether und 200 ml Diethylether eingerührt wobei das Produkt ausfällt. Das Produkt wird abgesaugt und im Exsiccator getrocknet.
Ausbeute: 17.4 g
R_{f} = 0.63 (Dichlormethan/Methanol/Triethylamin 100:5:1)

### Beispiel 61

### Herstellung von N-((4-Methoxyphenyl)-diphenylmethylamino)ethyl-N-((1-(1,2,4)-triazolyl)acetyl)glycin Mmt-Aeg(Triaz)-OH

5.13 g N-((4-Methoxyphenyl)-diphenylmethylamino)ethyl-N-((1-(1,2,4)triazolyl)acetyl)glycinmethylester werden in einer Mischung aus 10 ml Dioxan, 10 ml Methanol, 80 ml Wasser und 13.9 ml Triethylamin gelöst. Die klare Lösung läßt man 48 h bei Raumtemperatur stehen. Danach ist das Ausgangsmaterial verseift und die Lösung wird im Vakuum am Rotationsverdampfer zur Trockene eingeengt. Der Rückstand wird noch zweimal mit wenig Toluol nachdestilliert Der Rückstand wird in einer Mischung aus je 20 ml Dioxan und Ethylacetat aufgenommen, von etwas Ungelöstem abfiltriert und das Filtrat in 500 ml Ether, der 1% Triethylamin enthält, eingerührt. Das ausgefallene Rohprodukt (4.02 g) wird an Kieselgel mit mit einem Stufengradienten (2-15 %) von Methanol/Ethylacetat(1:1) in Dichlormethan mit 1% Triethylamin in allen Lösungsmitteln chromatographisch gereinigt. Die das Produkt enthaltenden Fraktionen werden vereinigt und im Vakuum am Rotationsverdampfer zur Trockene eingeengt.
Ausbeute: 3.25 g
R_{f} = 0.27 (Dichlormethan/Methanol/Triethylamin 100:10:1)
MS(ES⁺): 500.2 (M+H)⁺

## Patentansprüche

1. Verbindung der Formel I 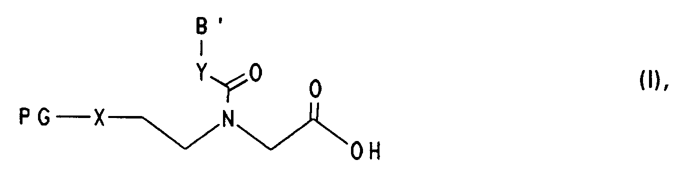 worin
PG für eine gegen schwache Säuren labile Aminoschutzgruppe vom Urethantyp oder vom Trityl-Typ,
X für NH, O oder S,
Y für CH₂, NH oder O, und
B' für in der Nucleotidchemie übliche Basen stehen, deren exocyclische Amino bzw. Hydroxygruppen durch geeignete bekannte Schutzgruppen geschützt sind, oder für Basenersatzverbindungen stehen,
sowie deren Salze.

2. Verbindung der Formel I gemäß Anspruch 1, worin
PG für eine gegen schwache Säuren labile Aminoschutzgruppe vom Urethantyp aus der Reihe 1-(1-Adamantyl)1-methylethoxycarbonyl (Adpoc), 1-(3,5-di-tert.-butylphenyl)1-methylethoxycarbonyl (t-Bumeoc) und 1-Methyl-1-(4-biphenyl)ethyloxycarbonyl (Bpoc) oder vom Trityl-Typ aus der Reihe Triphenylmethyl (Trt), (4-Methoxyphenyl)diphenylmethyl (Mmt), (4-Methylphenyl)diphenylmethyl (Mtt), Di-(4-methoxyphenyl)phenylmethyl (Dmt) und 9-(9-Phenyl)xanthenyl (Pixyl), und
B' für eine natürliche Nucleobase wie Adenin, Cytosin, Guanin, Thymin und Uracil oder eine unnatürliche Nucleobase, wie Purin, 2.6-Diaminopurin, 7-Deazaadenin, 7-Deazaguanin, N⁴N⁴-Ethanocytosin, N⁶N⁶-Ethano-2.6-diaminopurin, 5-Methylcytosin,5-(C₂-C₆)-Alkinyl-uracil, 5-(C₂-C₆)-Alkinylcytosin, 5-Fluor-uracil und Pseudoisocytosin, deren exocyclische Amino bzw. Hydroxygruppen durch geeignete bekannte Schutzgruppen geschützt sind, oder für Imidazol, Triazol oder Nitro-Imidazol stehen.

3. Verbindung der Formel I gemäß Anspruch 1 oder 2, worin
X für NH oder O,
Y für CH₂, und
B' für eine natürliche Nucleobase aus der Reihe Adenin, Cytosin, Guanin, Thymin und Uracil, oder eine unnatürliche Nucleobase aus der Reihe Purin, 2.6-Diaminopurin, 7-Deazaadenin, 7-Deazaguanin, N⁴N⁴-Ethanocytosin, N⁶N⁶-Ethano-2.6-diaminopurin, 5-Methylcytosin,5-(C₃-C₆)-Alkinyl-uracil, 5-(C₃-C₆)-Alkinyl-cytosin, 5-Fluor-uracil und Pseudoisocytosin, deren exocyclische Amino bzw. Hydroxygruppen durch die Benzoyl-, Isobutanoyl-, Acetyl-, Phenoxyacetyl-, 4-(t-Butyl)-benzoyl-, 4-(t-Butyl)-Phenoxyacetyl-, 4-(Methoxy)-benzoyl, 2-(4-Nitrophenyl)ethyl-oxycarbonyl-, 2-(2,4-Dinitrophenyl)ethyl-oxycarbonyl-, 9-Fluorenylmethoxycarbonyl-, Diphenylcarbamoyl- oder Formamidin-Schutzgruppe geschützt sind, und auch für Guanin durch eine Kombination von 2-N-Acetyl mit 6-O-Diphenylcarbamoyl geschützt sind, oder für Imidazol, Triazol oder Nitro-Imidazol stehen,

4. Verfahren zur Herstellung einer Verbindung der Formel I mit Y = CH₂ gemäß den Ansprüchen 1 bis 3, dadurch gekennzeichnet, daß man
a) eine Verbindung der Formel II 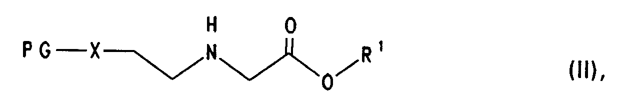 worin
PG und X wie in Anspruch 1 oder 2 definiert sind,
R¹ Wasserstoff oder eine Esterschutzgruppe bedeutet,
mit einer Verbindung der Formel III 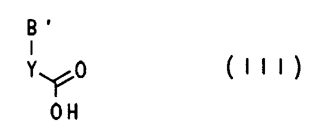 worin
B' wie oben definiert ist und
Y für CH₂ steht,
bei 0 - 45°C in einem geeigneten Lösungsmittel oder Mischungen dieser Lösungsmittel unter Verwendung eines in der Peptidchemie üblichen Kupplungsreagenzes zu einer Verbindung der Formel IV 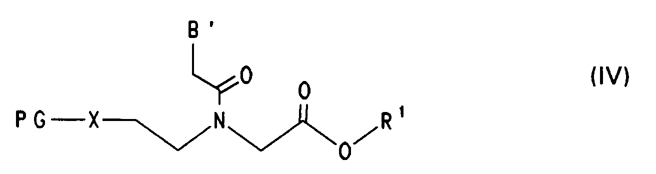 worin
PG, X, B' und R¹ wie oben definiert sind,
umsetzt und anschließend diese Verbindung durch Abspaltung der Esterschutzgruppe R¹ unter schwach alkalischen Bedingungen mit Alkalilauge, oder durch tertiäre Aminverbindungen in Wasser, oder auch enzymatisch mit Hilfe von Esterasen oder Lipasen bei 0 - 50°C in einem geeigneten Lösungsmittel oder Gemischen aus diesen Lösungsmitteln in eine Verbindung der Formel I überführt, oder
b) eine Verbindung der Formel II 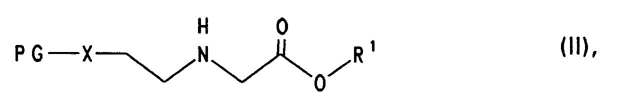 worin
PG und X wie oben definiert sind und
R¹ Wasserstoff oder eine temporäre Silylschutzgruppe bedeutet,
mit einer Verbindung der Formel V
B' - CH₂ - CO - R² (V),
worin
B' wie oben definiert ist und
R² Halogen oder den Rest eines Aktivesters bedeutet,
bei 0 - 40°C in einem geeigneten Lösungsmittel oder Mischungen dieser Lösungsmittel umsetzt, wobei gegebenenfalls der temporäre Schutz der Säurefunktion in der Verbindung der Formel II durch Umsetzung mit üblichen Silylierungsreagenzien und die Abspaltung dieser temporären Schutzgruppe - nach der Umsetzung mit der Verbindung der Formel V - durch Zugabe von Wasser oder Alkoholen zur Reaktionsmischung erfolgen kann, oder
c) eine Verbindung der Formel II 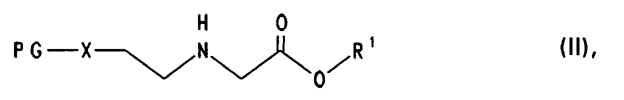 worin
PG und X wie oben definiert sind und
R¹ eine Esterschutzgruppe bedeutet,
mit einem Halogen-essigsäurederivat in einem geeigneten Lösungsmittel mit einer Hilfsbase zur Verbindung der Formel VI 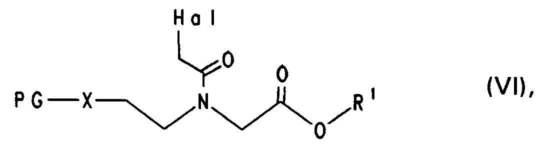 worin
Hal Cl, Br oder I bedeutet und
PG, X und R¹ wie oben definiert sind,
umsetzt,
diese Zwischenverbindung der Formel VI mit der gegebenenfalls geschützten Nucleobase B' und einer Hilfsbase in einem geeigneten Lösungsmittel zur Verbindung der Formel IV 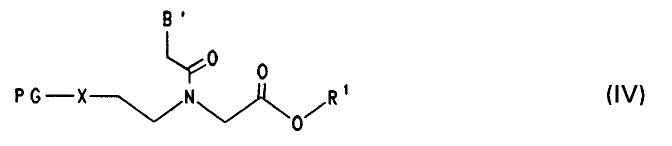 umsetzt,
danach diese Verbindung durch Abspaltung der Esterschutzgruppe R¹ mit Alkalilauge oder auch enzymatisch mit Hilfe von Esterasen oder Lipasen bei 0 - 50°C in einem geeigneten Lösungsmittel oder Gemischen aus diesen Lösungsmitteln in eine Verbindung der Formel I überführt.

5. Verfahren zur Herstellung einer Verbindung der Formel I mit Y = O oder NH gemäß den Ansprüchen 1 bis 3, dadurch gekennzeichnet, daß man eine Verbindung der Formel II 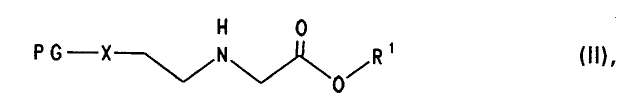 worin
PG und X wie oben definiert sind und
R¹ eine Esterschutzgruppe oder eine temporäre Esterschutzgruppe bedeutet, mit einer Verbindung der Formel VII
B' - Y - CO - R³ (VII),
worin
B' wie oben definiert ist,
Y O oder NH bedeutet und
R³ Cl, ONp oder ONSu bedeutet,
bei 0 - 40°C in einem geeigneten Lösungsmittel oder Mischungen dieser Lösungsmittel umsetzt und anschließend die Esterschutzgruppe R¹ mit Alkalilauge oder auch enzymatisch mit Hilfe von Esterasen oder Lipasen bei 0 - 50°C in einem geeigneten Lösungsmittel oder Gemischen aus diesen Lösungsmitteln abspaltet.

6. Verwendung der Verbindungen der Formel I gemäß den Ansprüchen 1 bis 3 zur Herstellung von PNA und PNA-/DNA-Hybriden.
